# EUROPEAN PATENT SPECIFICATION

(11) **EP 4 078 224 B1**
(45) Date of publication and mention of the grant of the patent: **18.11.2026**
(21) Application number: 20901401.8
(22) Date of filing: 16.12.2020
(51) Int. Cl.: B06B 1/02, H10W 70/09, H10W 70/60, A61B 8/00, H10W 70/00, H10W 70/652, H10W 72/00, H10W 72/20, H10W 72/90, H10W 74/10, H10W 90/10, H10W 90/00

(54) **METHODS AND APPARATUSES FOR PACKAGING ULTRASOUND-ON-CHIP DEVICES**
VERFAHREN UND VORRICHTUNGEN ZUM VERPACKEN VON ULTRASCHALL-ON-CHIP-VORRICHTUNGEN
PROCÉDÉS ET APPAREILS PERMETTANT DE CONDITIONNER DES DISPOSITIF À ULTRASONS SUR PUCE

(30) Priority: 17.12.2019 US 201962949318 P
(43) Date of publication of application: 26.10.2022
(73) Proprietor: BFLY Operations, Inc., Burlington, MA 01803 (US)
(72) Inventor: CHEN, Kailiang, Branford, Connecticut 06405 (US); SANCHEZ, Nevada J., Guilford, Connecticut 06437 (US); LIU, Jianwei, Fremont, California 94539 (US)
(74) Representative: Carroll, Christopher P.
(86) International application number: PCT/US2020/065330
(87) International publication number: WO 2021/126992

(56) References cited:
- WO-A1-2020/160002
- US-A1- 2017 207 214
- US-A1- 2019 142 387
- US-A1- 2019 142 387
- ATTARZADEH HOURIEH ET AL: "Stacking integration methodologies in 3D IC for 3D ultrasound image processing application: A stochastic flash ADC design case study", 2015 IEEE INTERNATIONAL SYMPOSIUM ON CIRCUITS AND SYSTEMS (ISCAS), IEEE, 24 May 2015 (2015-05-24), pages 1266 - 1269, XP033183403, DOI: 10.1109/ISCAS.2015.7168871
- ATTARZADEH HOURIEH ET AL: "Design and Analysis of a Stochastic Flash Analog-to-Digital Converter in 3D IC technology for integration with ultrasound transducer array", MICROELECTRONICS JOURNAL, vol. 48, 24 December 2015 (2015-12-24), pages 39 - 49, XP029384120, ISSN: 0026-2692, DOI: 10.1016/J.MEJO.2015.11.007

## Description

### RELATED APPLICATIONS

This application claims priority to U.S. Provisional Patent Application Serial No. 62/949,318, filed December 17, 2019, entitled "METHODS AND APPARATUSES FOR PACKAGING ULTRASOUND-ON-CHIP DEVICES".

### FIELD

Generally, the technology described herein relates to ultrasound devices. Aspects of the disclosure relate to an ultrasound-on-chip device and to a method.

### BACKGROUND

Ultrasound devices may be used to perform diagnostic imaging and/or treatment, using sound waves with frequencies that are higher with respect to those audible to humans. Ultrasound imaging may be used to see internal soft tissue body structures, for example to find a source of disease or to exclude any pathology. When pulses of ultrasound are transmitted into tissue (e.g., by using an ultrasound imaging device), sound waves are reflected off the tissue, with different tissues reflecting varying degrees of sound. These reflected sound waves may then be recorded and displayed as an ultrasound image to the operator. The strength (amplitude) of the sound signal and the time it takes for the wave to travel through the body provide information used to produce the ultrasound image. Many different types of images can be formed using ultrasound devices, including real-time images. For example, images can be generated that show two-dimensional cross-sections of tissue, blood flow, motion of tissue over time, the location of blood, the presence of specific molecules, the stiffness of tissue, or the anatomy of a three-dimensional region.

Attarzadeh Hourieh et al.: "Stacking integration methodologies in 3D IC for 3D ultrasound image processing application: A stochastic flash ADC design case study", 2015 IEEE International Symposium on Circuits And Systems (ISCAS), IEEE, 24 May 2015, pages 1266-1269, describes a stochastic flash analog-to-digital converter (ADC), implemented in a three-dimensional stacked technology. Due to vertical stacking, the 3D technology reduces the ADC footprint area, and a power consumption benefit for the 3D ADC is demonstrated compared with the 2D design. An all digitally synthesized stochastic 3D ADC was implemented using 130 nm GlobalFoundries device technology and Tezzaron through-silicon-vias (TSV) technology. Different TSV insertion methods were used and compared. A 20% improvement in power consumption is observed in the 3D implementation compared with the 2D counterpart. Thanks to the vertical stacking dies, a 40% footprint reduction is achieved in the 3D implementation. Two different integration topologies for 3D ADC and an ultrasound transducer arrays are considered and compared. Either the circuit dies are facing up, resulting in a face-to-face bonding between the transducer arrays and the three dimensional integrated circuit (3D IC), or the 3D IC dies are facing down, resulting in a face-to-back connection. The simulation results show that the 3D face-up integration suppresses the TSV coupling noise to the analog input by around 10 dB.

Attarzadeh Hourieh et al.: "Design and Analysis of a Stochastic Flash Analog-to-Digital Converter in 3D IC technology for integration with ultrasound transducer array", Microelectronics Journal, vol. 48, 24 December 2015, pages 39-49, discloses a stochastic flash analog-to-digital converter (ADC), implemented in a three-dimensional stacked technology. Due to vertical stacking, the 3D technology reduces the ADC footprint area, and a power consumption benefit for the 3D ADC is demonstrated compared with the 2D design. An all digitally synthesized stochastic 3D ADC was implemented using 130 nm GlobalFoundries device technology and Tezzaron through-silicon-vias (TSV) technology. Different TSV insertion methods were used and compared. A 20% improvement in power consumption is observed in the 3D implementation compared with the 2D counterpart. Thanks to the vertical stacking dies, a 40% footprint reduction is achieved in the 3D implementation. Two different integration topologies for 3D ADC and an ultrasound transducer arrays are considered and compared. Either the circuit dies are facing up, resulting in a face-to-face bonding between the transducer arrays and the three dimensional integrated circuit (3D IC), or the 3D IC dies are facing down, resulting a face-to-back connection. The simulation results show that the 3D face-up integration suppresses the TSV coupling noise to the analog input by around 10 dB.

US 2019/142387 A1 describes an ultrasound device including a first die that includes an ultrasonic transducer, a first application-specific integrated circuit (ASIC) that is bonded to the first die and includes a pulser, and a second ASIC in communication with the second ASIC that includes integrated digital receive circuitry. The first ASIC may be bonded to the second ASIC and the second ASIC may include analog processing circuitry and an analog-to-digital converter. In this case, the second ASIC may include a through-silicon via (TSV) facilitating communication between the first ASIC and the second ASIC. SERDES circuitry facilitates communication between the first ASIC and the second ASIC and the first ASIC includes analog processing circuitry and an analog-to-digital converter. The technology node of the first ASIC is different from the technology node of the second ASIC.

### SUMMARY

According to one aspect of the application, an ultrasound-on-chip device is provided as defined in claim 1.

In some embodiments, the ultrasound-on-chip device further includes ultrasonic transducers in the second integrated circuit substrate coupled to the second integrated ultrasound circuitry.

In some embodiments, the first integrated ultrasound circuitry of the first integrated circuit substrate comprises digital receive circuitry and the second integrated ultrasound circuitry of the second integrated circuit substrate comprises a pulser, a receive switch, analog receive circuitry, and an analog-to-digital converter. In some embodiments, the first integrated ultrasound circuitry of the first integrated circuit substrate comprises digital receive circuitry and the second integrated circuit substrate comprises an ultrasound transducer and the second integrated ultrasound circuitry comprises a pulser, a receive switch, analog receive circuitry, and an analog-to-digital converter.

In some embodiments, the first integrated ultrasound circuitry of the first integrated circuit substrate comprises digital receive circuitry, the second integrated circuit substrate comprises a first device and a second device bonded together, the first device comprises an ultrasound transducer, and the second integrated ultrasound circuitry is on the second device and comprises a pulser, a receive switch, analog receive circuitry, and an analog-to-digital converter. In some embodiments, the analog receive circuitry comprises one or more analog amplifiers, one or more analog filters, analog beamforming circuitry, analog dechirp circuitry, analog quadrature demodulation (AQDM) circuitry, analog time delay circuitry, analog phase shifter circuitry, analog summing circuitry, analog time gain compensation circuitry, and/or analog averaging circuitry. In some embodiments, the digital receive circuitry comprises one or more digital filters, digital beamforming circuitry, digital quadrature demodulation (DQDM) circuitry, averaging circuitry, digital dechirp circuitry, digital time delay circuitry, digital phase shifter circuitry, digital summing circuitry, digital multiplying circuitry, requantization circuitry, waveform removal circuitry, image formation circuitry, backend processing circuitry and/or one or more output buffers.

In some embodiments, the ultrasound-on-chip device includes a solder ball coupled to the first integrated circuit substrate. In some embodiments, the ultrasound-on-chip device includes a printed circuit substrate (PCB), and wherein the first integrated circuit substrate is disposed between the PCB and the second integrated circuit substrate.

In some embodiments, a communication link between the first integrated circuit substrate and a PCB is implemented through the first conductive pillar. In some embodiments, a communication link between the second integrated circuit substrate and a PCB is implemented through the second conductive pillar.

In some embodiments, the ultrasound-on-chip device is a portion of a wearable ultrasound-on-chip device. In some embodiments, the ultrasound-on-chip device is a portion of an ultrasound patch.

In some embodiments, the ultrasound-on-chip device further comprises a fourth conductive pillar, the first redistribution layer is coupled between the second conductive pillar and the fourth conductive pillar, and the second redistribution layer is coupled between the fourth conductive pillar and the second integrated circuit substrate or a contact on the second integrated circuit substrate. The first redistribution layer is a multilayer redistribution layer.

According to a further aspect of the application, a method is provided as defined in claim 8.

Further embodiments of the ultrasound-on-chip device and the method are defined in the dependent claims.

### BRIEF DESCRIPTION OF THE DRAWINGS

Various aspects and embodiments will be described with reference to the following exemplary and non-limiting figures. It should be appreciated that the figures are not necessarily drawn to scale. Items appearing in multiple figures are indicated by the same or a similar reference number in all the figures in which they appear.
FIGs. 1-26 illustrate cross-sections of an exemplary ultrasound-on-chip device during packaging, in accordance with certain embodiments described herein;
FIG. 27 illustrates a cross-section of another exemplary packaged ultrasound-on-chip device, in accordance with certain embodiments described herein;
FIGs. 28-29 illustrate cross-sections of another exemplary packaged ultrasound-on-chip device, in accordance with certain embodiments described herein;
FIG. 30 illustrates a cross-section of an exemplary ultrasound-on-chip device, in accordance with certain embodiments described herein;
FIG. 31 illustrates a cross-section of an exemplary ultrasound-on-chip device, in accordance with certain embodiments described herein;
FIG. 32 illustrates a cross-section of an exemplary ultrasound-on-chip device, in accordance with certain embodiments described herein
FIG. 33 illustrates a functional block diagram of an exemplary ultrasound-on-chip device, in accordance with certain embodiments described herein;
FIG. 34 illustrates a functional block diagram of another exemplary ultrasound-on-chip device, in accordance with certain embodiments described herein;
FIG. 35 illustrates a functional block diagram of an exemplary ultrasound device, in accordance with certain embodiments described herein;
FIG. 36 illustrates a schematic diagram of a side view of the ultrasound device of FIG. 35, in accordance with certain embodiments described herein;
FIG. 37 illustrates a schematic diagram of a top view of the ultrasound device of FIG. 35, in accordance with certain embodiments described herein;
FIG. 38 illustrates a schematic diagram of a bottom view of the ultrasound device of FIG. 35, in accordance with certain embodiments described herein;
FIG. 39 illustrates a top view of the ultrasound device of FIG. 35, in accordance with certain embodiments described herein;
FIG. 40 illustrates a top view of another ultrasound device, in accordance with certain embodiments described herein; and
FIG. 41 illustrates an example process for packaging an ultrasound-on-chip device, in accordance with certain embodiments described herein.

### DETAILED DESCRIPTION

Conventional ultrasound systems are large, complex, and expensive systems that are typically only purchased by large medical facilities with significant financial resources. Recently, less costly and less complex ultrasound imaging devices have been introduced. Such imaging devices may include ultrasonic transducers and ultrasound circuitry integrated onto one or more semiconductor dies. Ultrasound circuitry may refer to circuitry involved in driving ultrasonic transducers to transmit ultrasound waves and circuitry involved in receiving and processing ultrasound waves. Aspects of such ultrasound-on-chip devices (where "ultrasound-on-chip" does not preclude the device including two or more chips including ultrasonic transducers and/or integrated ultrasound circuitry) are described in U.S. Patent Application No. 15/415,434 titled "UNIVERSAL ULTRASOUND DEVICE AND RELATED APPARATUS AND METHODS," filed on January 25, 2017 and published as U.S. Pat. Publication No. 2017/0360397 A1 (and assigned to the assignee of the instant application).

Some implementations of ultrasound-on-chip devices may include integrated ultrasound transmit circuitry and integrated ultrasound receive circuitry implemented in the same device (e.g., die). The integrated transmit circuitry and integrated receive circuitry may be, for example, complementary metal-oxide-semiconductor (CMOS) circuitry. The integrated transmit circuitry may be configured to drive ultrasonic transducers to emit pulsed ultrasonic signals into a subject, such as a patient. The integrated transmit circuitry may include integrated analog circuitry such as pulsers. The pulsed ultrasonic signals may be back-scattered from structures in the body, such as blood cells or muscular tissue, to produce echoes that return to the ultrasonic transducers. These echoes may then be converted into electrical signals by the transducer elements. The integrated receive circuitry may be configured to convert the electrical signals representing the received echoes into ultrasound data that can, for example, be formed into an ultrasound image. The integrated receive circuitry may include integrated analog circuitry, such as analog receive circuitry and analog-to-digital converters (ADCs), and integrated digital circuitry, such as image formation circuitry.

The inventors have recognized that, in certain embodiments, it may be helpful to implement ultrasound transducers, analog portions of the integrated transmit circuitry (e.g., pulsers), and analog portions of the integrated receive circuitry (e.g., amplifiers and ADCs) in one device (e.g., an application-specific integrated circuit (ASIC)), and to implement digital portions of the integrated receive circuitry (e.g., image formation circuitry) in another device (e.g., an ASIC). Alternatively, in some embodiments, the ultrasound transducers may be implemented in one device, the analog portions of the integrated transmit and the analog portions of the integrated receive circuitry may be implemented in another device (e.g., an ASIC), and the two devices may be bonded together. Either embodiment may allow the device having the integrated analog circuitry to be implemented in a different technology node than the device having the integrated digital circuitry. In some embodiments, any digital transmit circuitry may be split between the devices, or implemented entirely on one or the other of the devices. As will be described below, the integrated analog circuitry may benefit from implementation in a less advanced (larger) technology node than the integrated digital circuitry, and the integrated digital circuitry may benefit from implementation in a more advanced (smaller) technology node than the integrated analog circuitry.

To drive the ultrasonic transducers, the inventors have recognized that pulsers may benefit from operating at high voltages that are approximately equal to or greater than 10 V, such as 10 V, 20 V, 30 V, 40 V, 50 V, 60 V, 70 V, 80 V, 90 V, 100 V, 200 V, or >200 V, or any value between 10 V and 300 V. Increasingly higher voltage levels of electronic signals outputted to ultrasonic transducers by the integrated transmit circuitry may correspond to higher pressure levels of acoustic signals outputted by the ultrasonic transducers. High pressure levels may be helpful for emitting acoustic signals into a patient, as pressure levels of acoustic signals are attenuated as they travel deeper into a patient. High pressure levels may also be necessary for certain types of ultrasound imaging such as tissue harmonic imaging. Circuit devices capable of operating at acceptably high voltage levels may only be available in sufficiently large technology nodes such as 65 nm, 80 nm, 90 nm, 110 nm, 130 nm, 150 nm, 180 nm, 220 nm, 240 nm, 250 nm, 280 nm, 350 nm, 500 nm, > 500 nm, etc.

Furthermore, when the amplifiers and ADCs are in the same device as the pulsers, the amplifiers and ADCs may receive weak signals from the ultrasonic transducers (in some embodiments, through bonds between two devices), amplify them, and digitize them. Tight coupling (e.g., low-resistance paths) between the device having the integrated analog circuitry and the device having the integrated digital circuitry may therefore not be necessary because the digitized signals outputted by analog-to-digital converters in the integrated analog circuitry to the device having the integrated digital circuitry may be resilient to attenuation and noise. In some embodiments, a high-speed communication link such as a serial-deserializer (SERDES) link may facilitate communication between the device having the integrated analog circuitry and the device having the integrated digital circuitry.

It may be helpful for the integrated digital circuitry, which may perform digital receive operations, to operate at low voltages that are approximately equal to or lower than, for example, 1.8 V, such as 1.8 V, 1.5 V, 1 V, 0.95 V, 0.9 V, 0.85 V, 0.8 V, 0.75 V, 0.7 V, 0.65 V, 0.6 V, 0.55 V, 0.5 V, and 0.45 V. The integrated digital circuitry may be densely integrated in order to increase its parallel computing power and may consume a significant portion (e.g., half) of the ultrasound device's power. Scaling the operating voltage of the integrated receive circuitry down by a factor N (where N>1) can reduce the power consumption by a factor N^{x} (where x≥1), such as N². Circuit devices capable of operating at acceptably low voltage levels may, in some embodiments, only be available in technology nodes such as 90 nm, 80 nm, 65 nm, 55 nm, 45 nm, 40 nm, 32 nm, 28 nm, 22 nm, 20 nm, 16 nm, 14 nm, 10 nm, 7 nm, 5 nm, 3 nm, etc. Furthermore, the inventors have recognized that it may be beneficial for the integrated digital circuitry to include smaller devices, for example sizes provided by technology nodes such as 90 nm, 80 nm, 65 nm, 55 nm, 45 nm, 40 nm, 32 nm, 28 nm, 22 nm, 20 nm, 16 nm, 14 nm, 10 nm, 7 nm, 5 nm, 3 nm, etc.), to increase the number of devices that can be included in a die of a given size, and thereby increase the processing (e.g., data conversion and image formation) capability of the integrated digital circuitry.

The inventors have recognized features that may be helpful for packaging ultrasound-on-chip devices having a first device that includes ultrasonic transducers, analog portions of the integrated transmit circuitry, and analog portions of the integrated receive circuitry and a second device that includes digital portions of the integrated receive circuitry, such that the packaged ultrasound-on-chip device is sufficiently small in size to form the core of a wearable ultrasound device. The wearable ultrasound device may be in the form-factor of an ultrasound patch or some other form-factor that can couple to a subject. This packaging may include a vertical stack of the two devices packaged with integrated fan-out packaging. Such packaging may include conductive pillars and redistribution layers that fan-out interconnect and thereby facilitate communication between the first device in the ultrasound-on-chip device and an external device (e.g., a printed circuit board (PCB)), between the second device in the ultrasound-on-chip device and the external device, and/or between the two devices in the ultrasound-on-chip device. Example benefits of such packaging compared with other packaging methods (e.g., wirebonding) include lower parasitic inductance and resistance, higher efficiency, less heating, higher packaging throughput, improved packaging reliability, and more compact size.

As referred to herein in the specification and claims, a device including a specific type of circuitry should be understood to mean that the device includes only that specific type of circuitry or that the device includes that specific type of circuitry and another type/other types of circuitry. For example, if an ultrasound device includes a second device and a third device, where the second device includes "integrated transmit circuitry" or "the integrated transmit circuitry" and the third device includes "integrated receive circuitry" or "the integrated receive circuitry," this may mean that the second device includes all the integrated transmit circuitry in the ultrasound device, the second device includes a portion of the integrated transmit circuitry in the ultrasound device, the third device includes all the integrated receive circuitry in the ultrasound device, and/or the third device includes a portion of the integrated receive circuitry in the ultrasound device. Furthermore, the second device may include only integrated transmit circuitry or other types of circuitry. For example, the second device may include both integrated transmit circuitry and integrated receive circuitry. Furthermore, the third device may include only integrated receive circuitry or other types of circuitry. For example, the third device may include both integrated receive circuitry and integrated transmit circuitry.

It should be appreciated that the embodiments described herein may be implemented in any of numerous ways. Examples of specific implementations are provided below for illustrative purposes only. It should be appreciated that these embodiments and the features/capabilities provided may be used individually, all together, or in any combination of two or more, as aspects of the technology described herein are not limited in this respect.

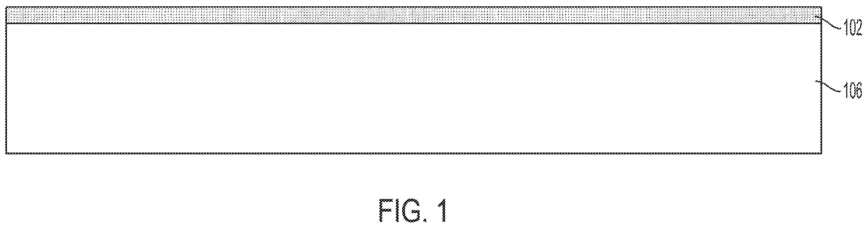

FIGs. 1-26 illustrate cross-sections of an exemplary ultrasound-on-chip device during packaging, in accordance with certain embodiments described herein. FIG. 1 illustrates a carrier substrate 106 and an insulating layer 102 coupled to the carrier substrate 106. The carrier substrate 106 may include, for example, glass. The insulating layer 102 may include, for example, a polymer that can be patterned with light exposure and developed, such as polyimide, polybenzoxazole (PBO), or benzocyclobutene (BCB). In some embodiments, a release layer (including, for example, light-to-heat-conversion (LTHC) coating material) may be coupled between the insulating layer 102 and the carrier substrate 106.

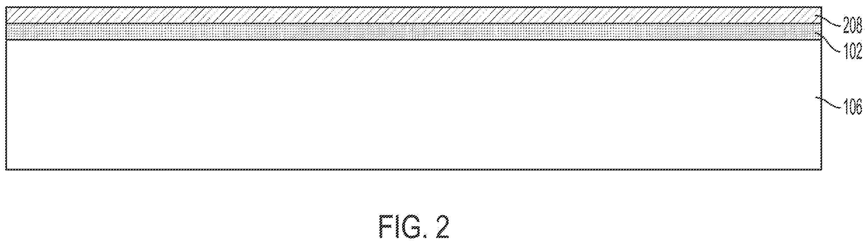

In FIG. 2, a conductive layer 208 is formed on the insulating layer 102. The conductive layer 208 may be formed, for example, using physical vapor deposition (PVD) or sputtering. The conductive layer 208 may include a metal, for example, copper, or in some embodiments, the conductive layer 208 may include two metal layers, such as a titanium layer coupled to the insulating layer 102 and a copper layer coupled to the titanium layer.

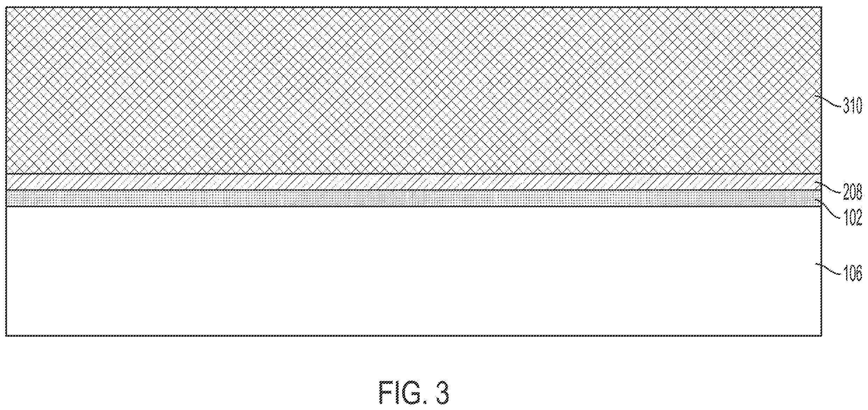

In FIG. 3, a resist layer 310 is formed on the conductive layer 208. For example, the resist layer 310 may include photoresist.

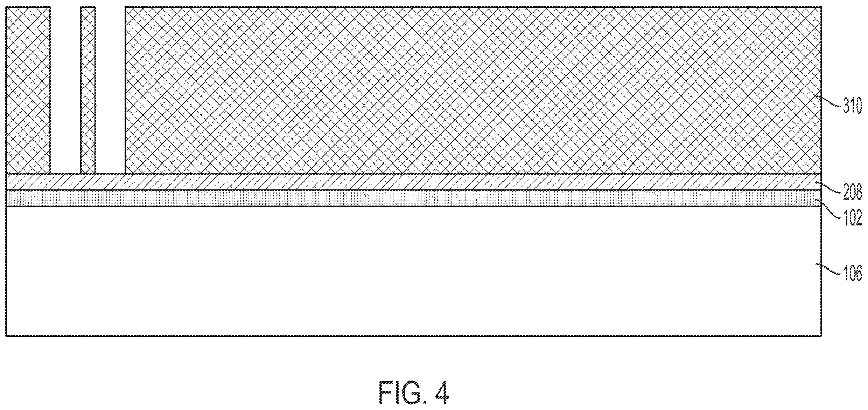

In FIG. 4, openings 404 are formed in the resist layer 310. For example, light exposure through a lithography mask followed by development may create the openings 404 in portions of the resist layer 310 that were exposed to light through the mask.

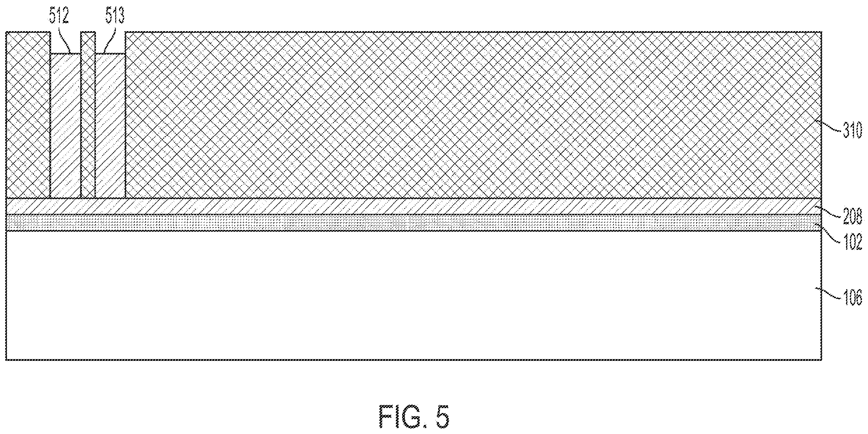

In FIG. 5, a conductive pillar 512 and a conductive pillar 513 are formed in the openings 404 in the resist layer 310 using electroplating. The conductive layer 208 may serve as a seed layer for the electroplating. The conductive pillars 512 and 513 may include the same material as the conductive layer 208. For example, the conductive pillars 512 and 513 may include a metal such as copper. It should be appreciated that while two conductive pillars 512 and 513 are shown, there may be more conductive pillars (e.g., tens or hundreds) arranged two-dimensionally with respect to a plane of the top surface of the structure.

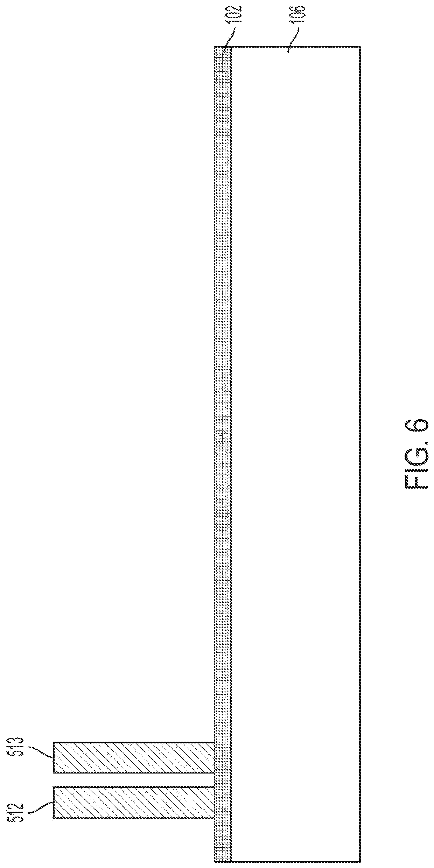

In FIG. 6, the resist layer 310 is removed. For example, a resist stripper may be used to remove the resist layer 310. Portions of the conductive layer 208 that were previously below unexposed portions of the resist layer 310 are also removed. For example, a selective, anisotropic etch may be used to remove portions of the conductive layer 208 not directly beneath the conductive pillars 512, 513, in which the material of the conductive layer 208 is etched faster than the material of the conductive pillars 512, 513.

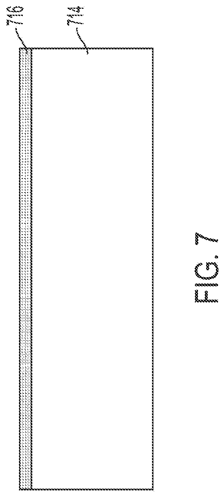

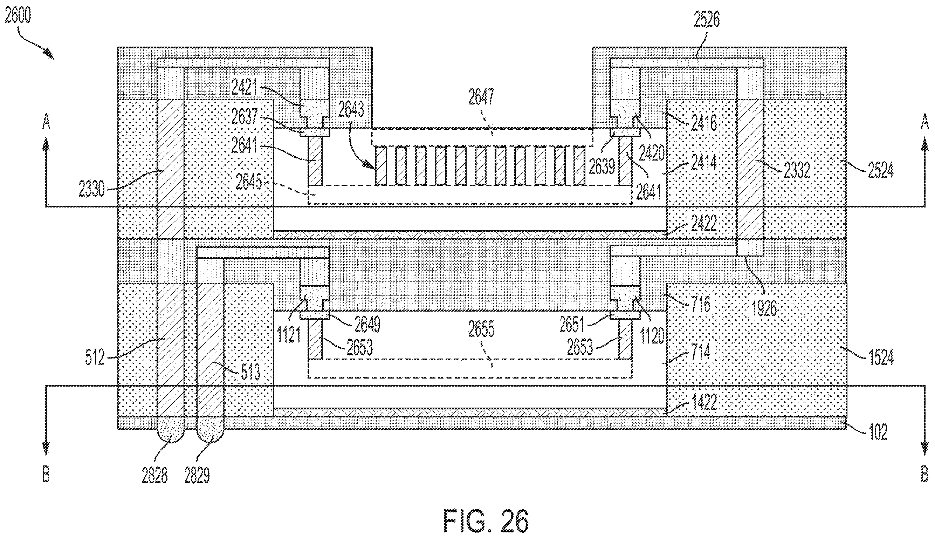

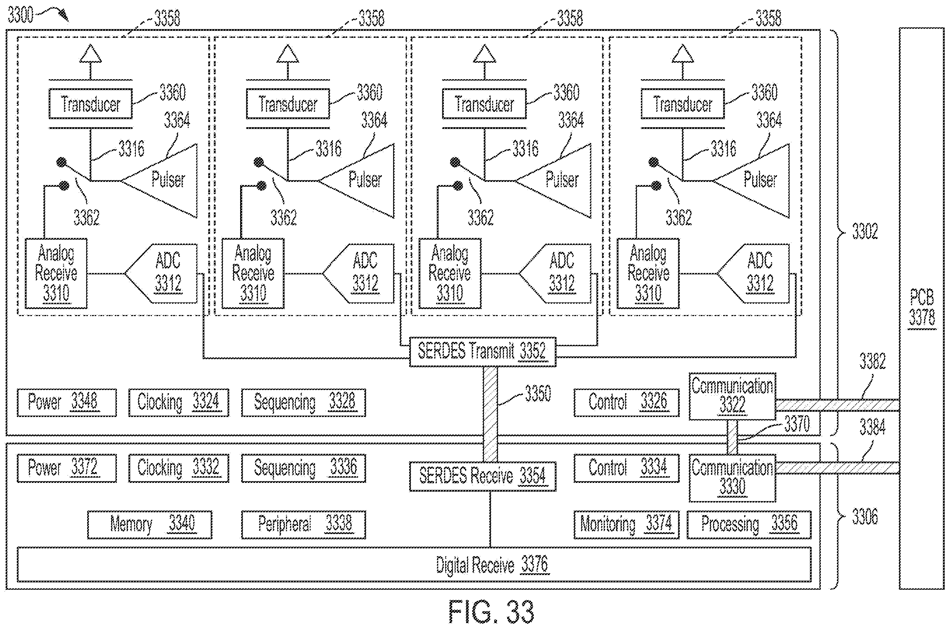

FIG. 7 illustrates a first integrated circuit substrate 714 coupled to an insulating layer 716. The first integrated circuit substrate 714 includes integrated ultrasound circuitry 2655 (shown in FIG. 26). Further description of the first integrated circuit substrate 714 may be found below with reference to FIGs. 33-34. It should be appreciated that the first integrated circuit substrate 714 may include two or more substrates bonded together.

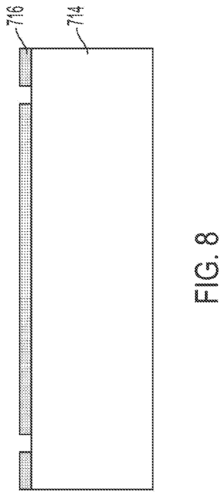

In FIG. 8, openings 834 are created in the insulating layer 716 (e.g., using photolithography of the kind described above with reference to FIGs. 3-6).

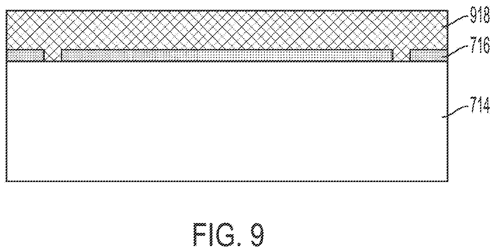

In FIG. 9, a resist layer 918 is formed on the insulating layer 716.

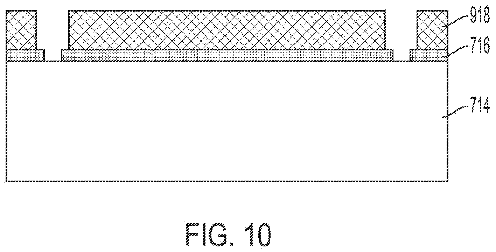

In FIG. 10, openings are created in the resist layer 918 (e.g., using photolithography of the kind described above), where the openings created in the resist layer 918 extend into the openings created in the insulating layer 716.

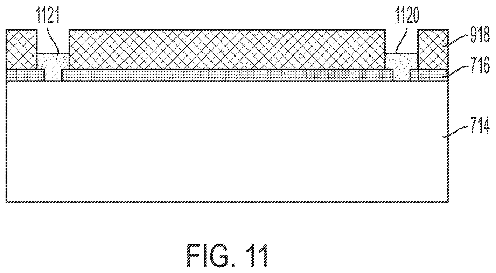

In FIG. 11, a contact 1120 and a contact 1121 are formed within the openings in the resist layer 918 and the insulating layer 716. For example, the contacts 1120 and 1121 may be formed by electroplating, and may include copper or a copper alloy. In some embodiments, an under-bump metallurgy layer (not shown in FIG. 11) may be formed between the contacts 1120 and 1121 and the first integrated circuit substrate 714. The contacts 1120 and 1121 may be electrically connected to integrated circuitry in the first integrated circuit substrate 714. For example, the contact 1120 and the contact 1121 may each be electrically connected to different portions of the integrated circuitry in the first integrated circuit substrate 714. It should be appreciated that while two contacts 1120 and 1121 are shown, there may be more contacts (e.g., tens or hundreds) arranged two-dimensionally with respect to a plane of the top surface of the structure.

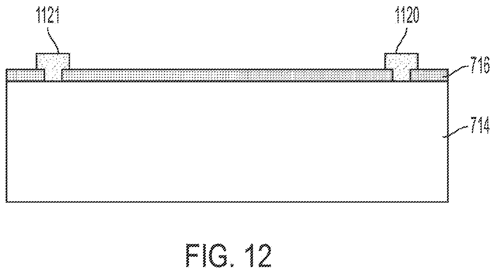

In FIG. 12, the resist layer 918 is removed (e.g., using a resist stripper).

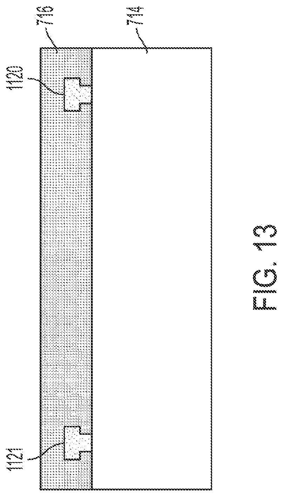

In FIG. 13, further insulating material is added to the insulating layer 716 to cover the contacts 1120 and 1121.

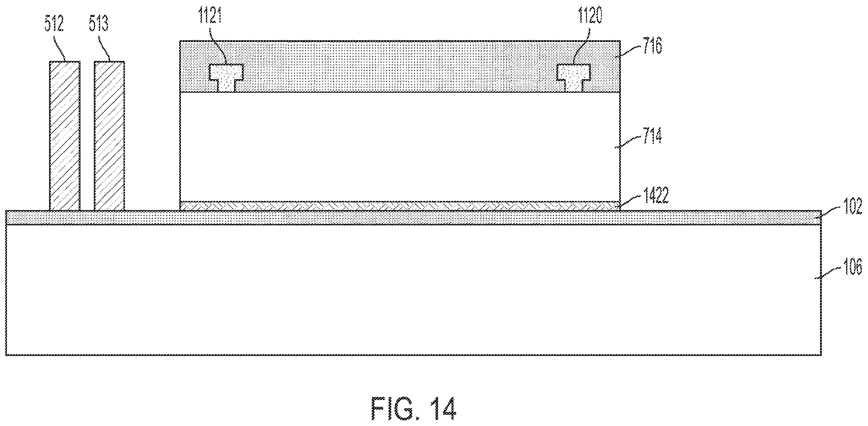

In FIG. 14, the first integrated circuit substrate 714 is coupled to the insulating layer 102 through a die-attach film 1422. The die-attach film 1422 may be coupled to the first integrated circuit substrate 714 before, after, or during any portion of the process illustrated in FIGs. 7-13.

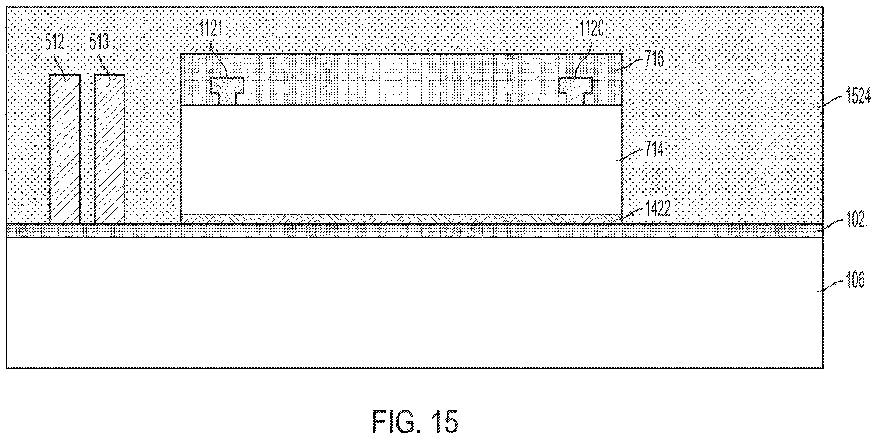

In FIG. 15, encapsulation 1524 is formed to encapsulate the first integrated circuit substrate 714, the insulating layer 716, the die-attach film 1422, and the conductive pillars 512 and 513. The encapsulation 1524 may include a molding compound, a molding underfill, an epoxy, and/or a resin. The top surface of the encapsulation 1524 extends above the top surfaces of the insulating layer 716 and the conductive pillars 512 and 513.

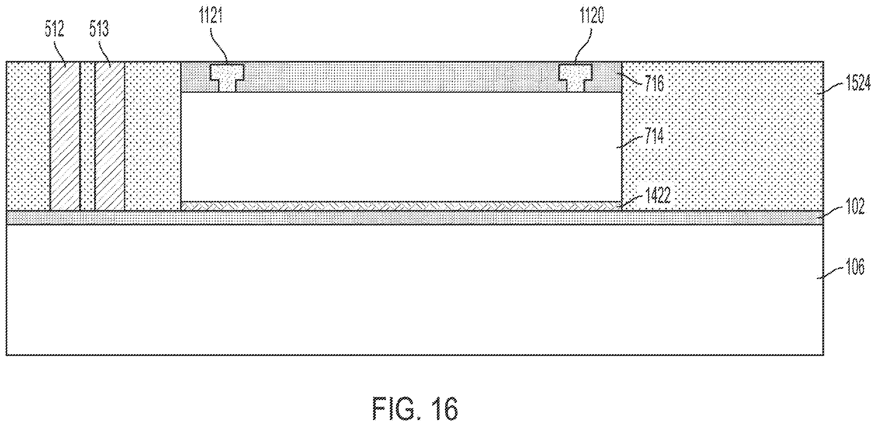

In FIG. 16, the top surfaces of the encapsulation 1524 and the insulating layer 716 are planarized until the top surfaces of the top surfaces of the conductive pillars 512 and 513 and the contacts 1120 and 1121 are exposed. For example, chemical mechanical planarization (CMP) may be used for the planarization.

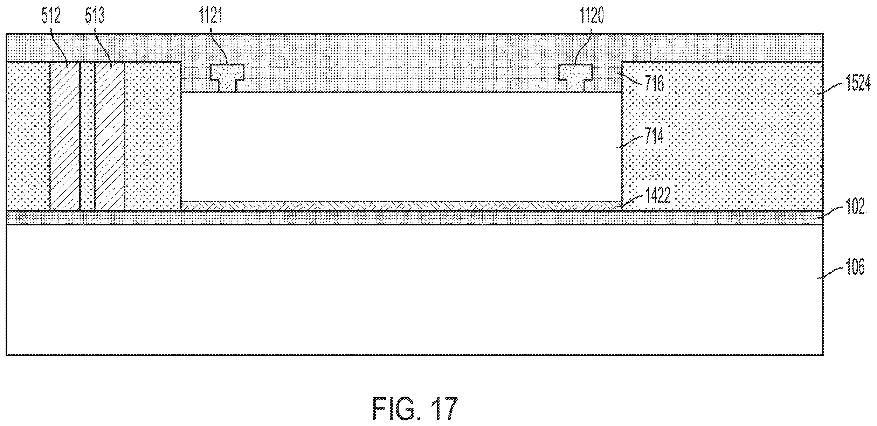

In FIG. 17, additional insulating material is added to the insulating layer 716, such that the insulating layer 716 covers the top surfaces of the contacts 1120 and 1121 and the conductive pillars 512 and 513.

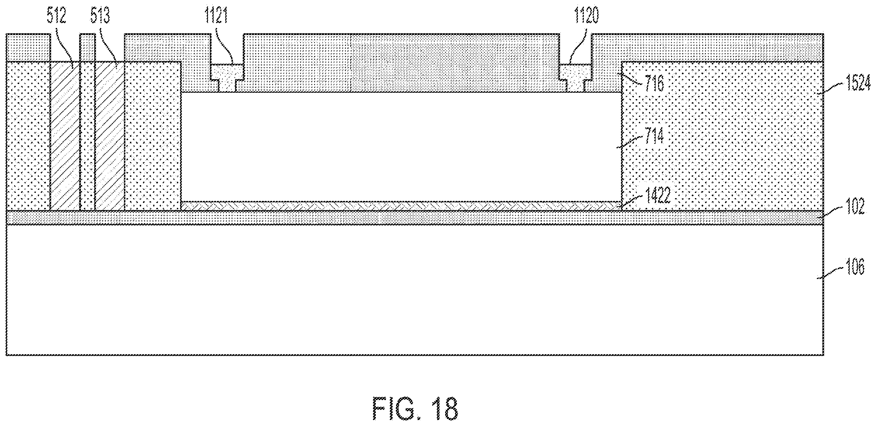

In FIG. 18, openings are created in the insulating layer 716 above the contacts 1120 and 1121 and the conductive pillars 512 and 513. For example, photolithography of the type described above with reference to FIGs. 3-6 may be used to create the openings.

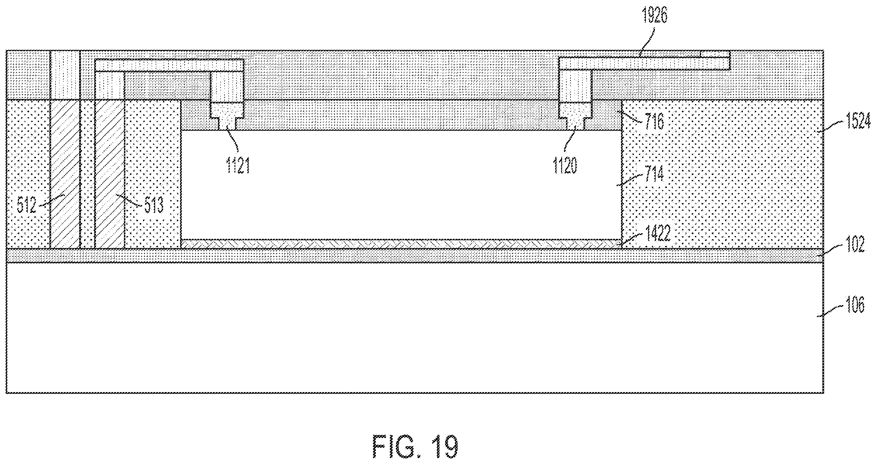

In FIG. 19, a redistribution layer (RDL) 1926 is formed above the conductive pillars 513 and 512, the contacts 1120 and 1121, and the insulating layer 716. The RDL 1926 extends through the openings formed in the insulating layer 716 to the conductive pillar 513, the conductive pillar 512, the contact 1121, and the contact 1120, and extends between the conductive pillar 513 and the contact 1121. Thus, the RDL 1926 may electrically connect the contact 1121 to the conductive pillar 513, and thereby electrically connect the conductive pillar 513 through the RDL 1926 and the contact 1121 to integrated circuitry in the first integrated circuit substrate 714. The RDL 1926 include metal traces and vias, may be formed using electroplating (including formation of a seed layer not shown), and may include metal such as aluminum, copper, tungsten, and/or alloys of these metals. The RDL 1926 may be formed by forming metal traces, vias, and insulating material in multiple steps. It should be appreciated that the RDL 1926 may include more portions than shown. For example, there may be more conductive pillars than illustrated to which the RDL 1926 may connect.

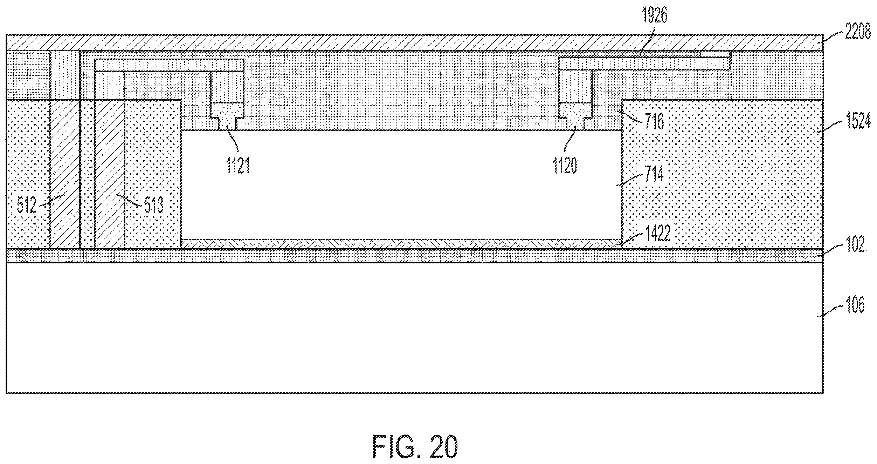

In FIG. 20, a conductive layer 2208 is formed on the insulating layer 716 and portions of the RDL 1926. Further description of the conductive layer 2208 may be found with reference to the previous description of the conductive layer 208.

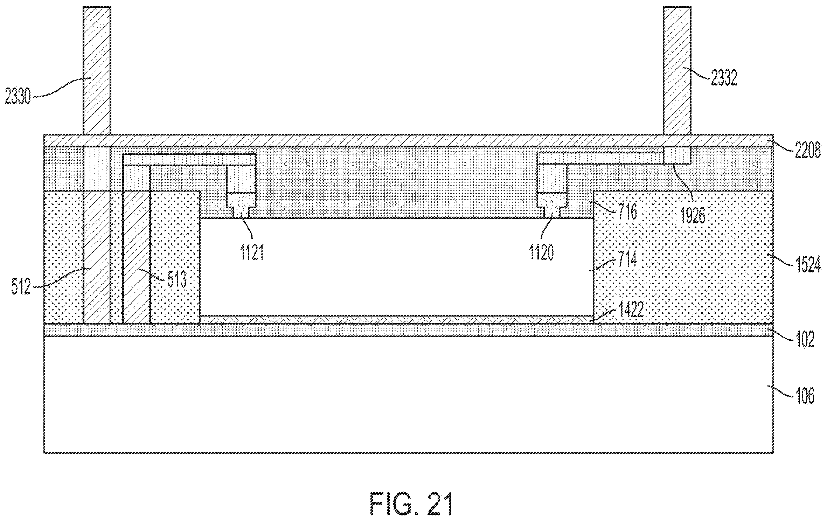

In FIG. 21, conductive pillars 2330 and 2332 are formed on the conductive layer 2208. Further description of the conductive pillars 2330 and 2332 may be found with reference to the previous description of the conductive pillars 512 and 513. The conductive layer 2208 may serve as a seed layer for electroplating the conductive pillars 2330 and 2332. The conductive pillar 2330 is electrically connected to a portion of the RDL 1926 that is electrically coupled to the conductive pillar 512. The conductive pillar 2332 is electrically connected to a portion of the RDL 1926 that is electrically coupled to the contact 1120. It should be appreciated that while two conductive pillars 2330 and 2332 are shown, there may be more conductive pillars (e.g., tens or hundreds) arranged two-dimensionally with respect to a plane of the top surface of the structure.

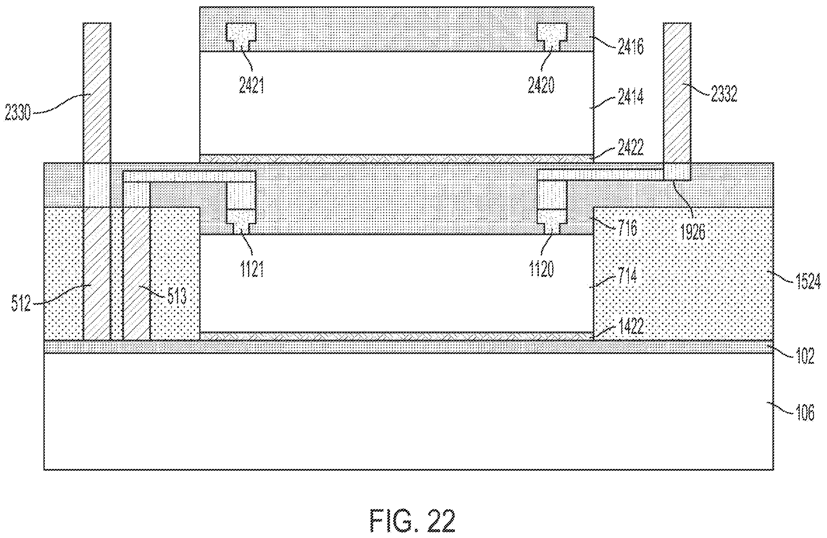

FIG. 22 illustrates a second integrated circuit substrate 2414, a die-attach film 2422, an insulating layer 2416, a contact 2421, and a contact 2420. The second integrated circuit substrate 2414 includes integrated ultrasound circuitry 2645 (shown in FIG. 26) and ultrasonic transducers 2647 (shown in FIG. 26). Further description of the second integrated circuit substrate 2414 may be found below with reference to FIGs. 33-34. It should be appreciated that the second integrated circuit substrate 2414 may include two or more substrates bonded together. The contacts 2420 and 2421 may be electrically connected to integrated circuitry in the second integrated circuit substrate 2414. It should be appreciated that while two contacts 2420 and 2421 are shown, there may be more contacts (e.g., tens or hundreds) arranged two-dimensionally with respect to a plane of the top surface of the structure. The second integrated circuit substrate 2414 is coupled to the insulating layer 716 through the die-attach film 2422. Further description of the die-attach film 2422, the insulating layer 2416, the contact 2420, and the contact 2421, and their fabrication may be found with reference to the previous description of the die-attach film 1422, the insulating layer 716, the contact 1120, and the contact 1121 in FIGs. 7-14. Portions of the conductive layer 2208 that are not below the conductive pillars 2330 and 2332 have also been removed. Further description of removing portions of the conductive layer 2208 may be found with reference to FIG. 6.

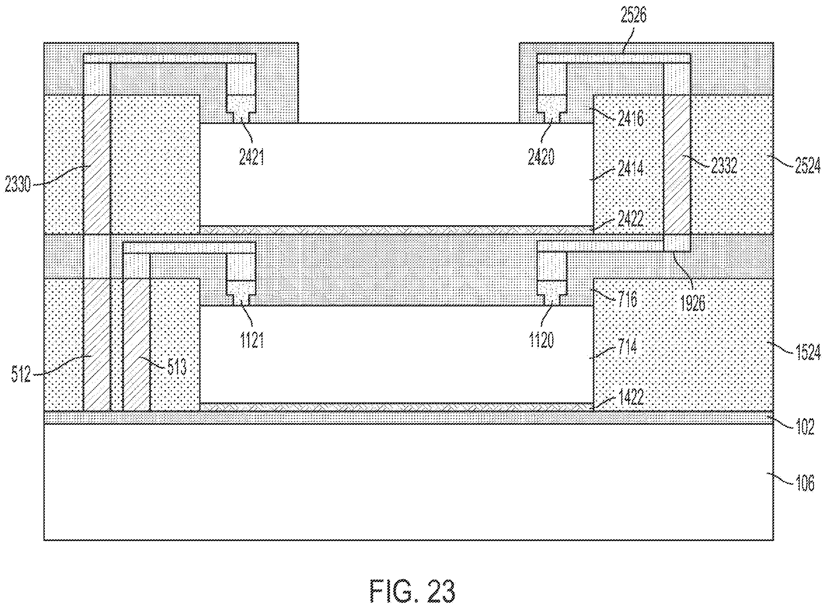

FIG. 23 illustrates encapsulation 2524 and RDL 2526. The RDL 2526 is formed between the conductive pillar 2330 and the contact 2421 and between the conductive pillar 2332 and the contact 2420. Thus, the RDL 2526 may electrically connect the conductive pillar 2330 through the contact 2421 to integrated circuitry in the second integrated circuit substrate 2414, and may electrically connect the conductive pillar 2332 through the contact 2420 to integrated circuitry in the second integrated circuit substrate 2414. Further description of the encapsulation 2524 and the RDL 2526 and their fabrication may be found with reference to the previous description of the encapsulation 1524 and the RDL 1926 and FIGs. 15-19. It should be appreciated that the RDL 2526 may include more portions than shown. For example, there may be more conductive pillars to which the RDL 2526 may connect.

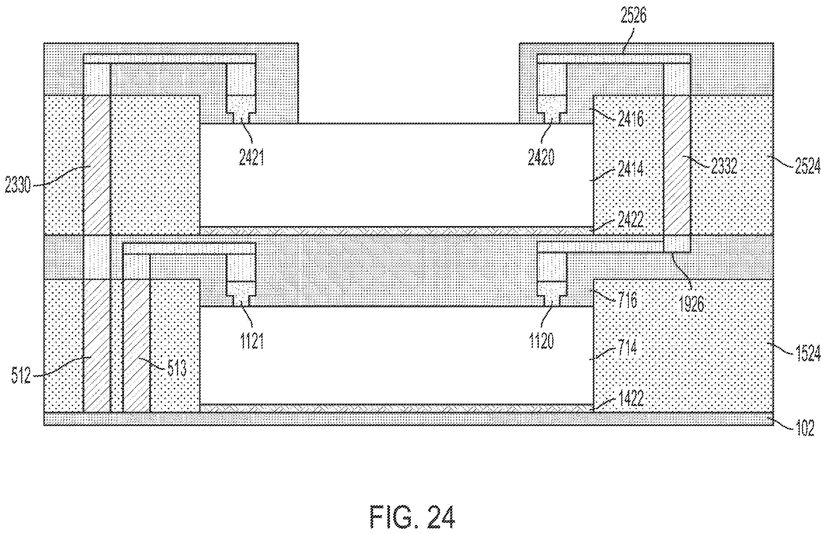

In FIG. 24 the carrier substrate 106 is detached from the insulating layer 102. In some embodiments, there may be a release layer (not shown) coupled between the insulating layer 102 and the carrier substrate 106. Projecting light (e.g., ultraviolet or laser) onto the release layer may decompose the release layer, causing the release layer and the carrier substrate 106 to detach from the insulating layer 102. The surface of the insulating layer 102 may also be cleaned to remove any residue. In some embodiments, other methods for removing the carrier substrate 106 may be used.

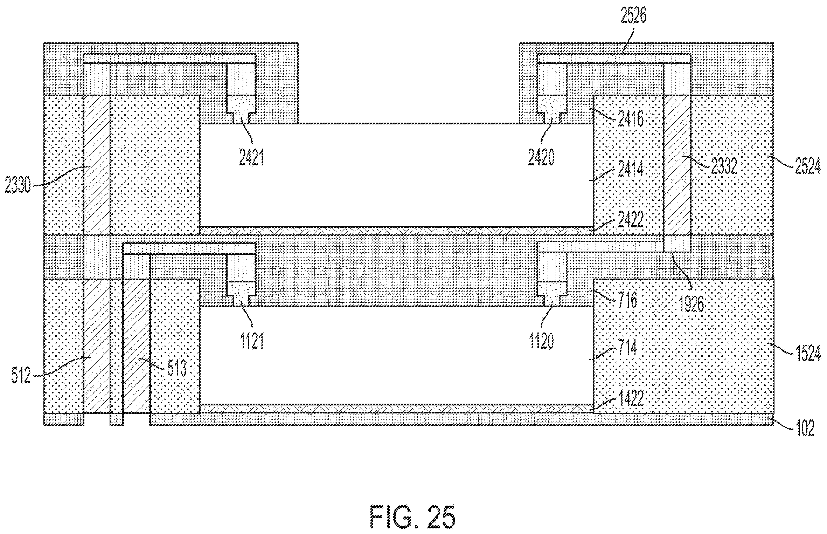

In FIG. 25, openings are created in the insulating layer 102 (e.g., using photolithography of the kind described above with reference to FIGs. 3-6) so as to expose the conductive pillars 512, 513.

In FIG. 26, a solder ball 2828 is coupled to the conductive pillar 512 through an opening in the insulating layer 102, and a solder ball 2829 is coupled to the conductive pillar 513 through an opening in the insulating layer. Thus, the solder ball 2828 may be electrically coupled to the conductive pillar 512 and the solder ball 2829 may be electrically coupled to the conductive pillar 513. In some embodiments, the solder balls 2828 and 2829 may be formed by electroplating. In some embodiments, other forms of electrical connectors (e.g., conductive pillars) may be formed in the openings. In some embodiments, an under-bump metallurgy layer (not shown in FIG. 26) may be formed between the solder balls 2828 and 2829 and the conductive pillars 512 and 513. It should be appreciated that while two solder balls 2828 and 2829 are shown, there may be more solder balls (e.g., tens or hundreds) arranged two-dimensionally with respect to a plane of the bottom surface of the structure.

FIG. 26 illustrates a packaged ultrasound-on-chip device 2600. The ultrasound-on-chip device 2600 includes two separate integrated circuit substrates, the first integrated circuit substrate 714 and the second integrated circuit substrate 2414, as well as packaging. The first integrated circuit substrate 714 and the second integrated circuit substrate 2414 may be considered to be arranged in a vertical stack. In some embodiments, a vertical stack may involve at least a portion of one integrated circuit substrate being above at least a portion of another integrated circuit substrate, and does not preclude other elements interposing between the two integrated circuit substrates. In some embodiments, a vertical stack may include one integrated circuit substrate substantially or entirely overlying another integrated circuit substrate. Such a configuration may represent the most compact design, in some embodiments, and may thus be beneficial for providing space savings and cost savings, as well as allowing for a smaller form factor of an overall device incorporating such a stacked device. The first integrated circuit substrate 714 may be considered to be on one level and the second integrated circuit substrate 2414 may be considered to be on another level. The packaging of the ultrasound-on-chip device includes the conductive pillars 512, 513, 2330, and 2332, the RDL 1926 and 2526, and the solder balls 2828 and 2829. In some embodiments, the solder balls 2828 and 2829 may be coupled to a printed circuit board (PCB). In some embodiments, the solder balls 2828 and 2829 may be coupled to a heat sink. In some embodiments, the solder balls 2828 and 2829 may be coupled to an interposer that is coupled to a PCB. In some embodiments, the interposer may also function as a heat sink. In some embodiments, the first integrated circuit substrate 714 may be disposed between a PCB and the second integrated circuit substrate 2414.

FIG. 26 further illustrates a bonding pad 2637, a bonding pad 2639, vias 2641, vias 2643, integrated ultrasound circuitry 2645, and ultrasonic transducers 2647 in the second integrated circuit substrate 2414, and a bonding pod 2649, a bonding pad 2651, vias 2653, and integrated ultrasound circuitry 2655 in the first integrated circuit substrate 714. It should be appreciated that FIG. 26 does not illustrate actual physical locations of these components within the first integrated circuit substrate 714 and the second integrated circuit substrate 2414. Rather, FIG. 26 is intended to illustrate how internal components of the first integrated circuit substrate 714 and the second integrated circuit substrate 2414 are electrically coupled to each other and to the packaging. In other words, FIG. 26 illustrates block diagrams, rather than physical diagrams, of the first integrated circuit substrate 714 and the second integrated circuit substrate 2414.

The bonding pad 2637 is coupled between the contact 2421 and a via 2641. The bonding pad 2639 is coupled between the contact 2420 and a via 2641. The vias 2641 are coupled between the integrated ultrasound circuitry 2645 and the bonding pads 2637 and 2639. The vias 2643 are coupled between the integrated ultrasound circuitry 2645 and the ultrasonic transducers 2647. There may be one via 2643 per ultrasonic transducer or group of ultrasonic transducers. The bonding pad 2649 is coupled between the contact 1121 and a via 2653. The bonding pad 2651 is coupled between the contact 1120 and a via 2653. The vias 2653 are coupled between the integrated ultrasound circuitry 2655 and the bonding pads 2649 and 2651. It should be appreciated that the integrated circuitry, bonding pads, and vias are be present in the first integrated circuit substrate 714 and second integrated circuit substrate 714 throughout the packaging process but are only shown in FIG. 26 for simplicity. Furthermore, integrated circuitry, bonding pads, and vias are present in other ultrasound devices described herein in the manner depicted in FIG. 26, but are not shown for simplicity.

For simplicity, only one via is shown, but each via shown may represent one or more layers of routing layers and vias between them. There may be more bonding pads for each integrated circuit substrate than shown. For example, there may be multiple rings of bonding pads (which may be flip-chip bonding pads) in the first integrated circuit substrate 714. Multiple routing layers may be helpful for routing between the bonding pads and the integrated ultrasound circuitry 2655. In some embodiments, there may not be multiple rings of bonding pads in the second integrated circuit substrate 2641 such that the center of the second integrated circuit substrate 2641 may be free for the ultrasonic transducers 2647. However, in some embodiments, either or both of the first integrated circuit substrate 714 and the second integrated circuit substrate 2414 may be multiple rings of bonding pads (e.g., flip-chip bonding pads) and/or multiple routing layers and vias. Further description of the integrated ultrasound circuitry 2645 and 2655 and the ultrasonic transducers 2547 may be found below with reference to FIGs. 33-34.

It should be appreciated that the packaging may facilitate electrical communication between an external electronic device (not shown) that is electrically coupled to the solder ball 2828, such as an electronic device on a PCB that is electrically coupled (in some embodiments, through a heat sink and/or interposer) to the solder ball 2828, and the integrated ultrasound circuitry 2645 in the second integrated circuit substrate 2414. In particular, electrical communication may occur through the solder ball 2828, the conductive pillar 512, the RDL 1926, the conductive pillar 2330, the RDL 2526, the contact 2421, the bonding pad 2637, the via 2641, and the integrated ultrasound circuitry 2645. It should also be appreciated that the packaging may facilitate electrical communication between an external electronic device that is electrically coupled to the solder ball 2829, such as an electronic device on a PCB that is electrically coupled (in some embodiments, through a heat sink and/or interposer) to the solder ball 2829, and the integrated ultrasound circuitry 2655 in the first integrated circuit substrate 714. In particular, electrical communication may occur through the solder ball 2829, the conductive pillar 513, the RDL 1926, the contact 1121, the bonding pad 2649, the via 2653, and the integrated ultrasound circuitry 2655. It should also be appreciated that the packaging may facilitate electrical communication between the integrated ultrasound circuitry 2655 in the first integrated substrate 714 and the integrated ultrasound circuitry 2645 in the second integrated circuit substrate 2414. In particular, electrical communication may occur through the integrated ultrasound circuitry 2655, the via 2653, the bonding pad 2651, the contact 1120, the RDL 1926, the conductive pillar 2332, the RDL 2526, the contact 2420, the bonding pad 2639, the via 2641, and the integrated ultrasound circuitry 2645. Thus, if a single PCB is coupled to the solder balls 2828 and 2829, the packaging may facilitate electrical communication between the single PCB and both the first and second integrated circuit substrates 714 and 2414 in the vertical stack, as well as facilitating electrical communication between the first and second integrated circuit substrates 714 and 2414 in the vertical stack. In some embodiments, electrical communication between the integrated ultrasound circuitry 2655 in the first integrated circuit substrate 714 and the integrated ultrasound circuitry 2645 in the second integrated circuit substrate 2414 may occur through the integrated ultrasound circuitry 2655, the via 2653, the bonding pad 2649, the contact 1121, the RDL 1926, the conductive pillar 513, the solder ball 2829, a PCB coupled to the solder balls 2828 and 2829, the solder ball 2828, the conductive pillar 512, the RDL 1926, the conductive pillar 2330, the RDL 2526, the contact 2421, the bonding pad 2637, the via 2641, and the integrated ultrasound circuitry 2645.

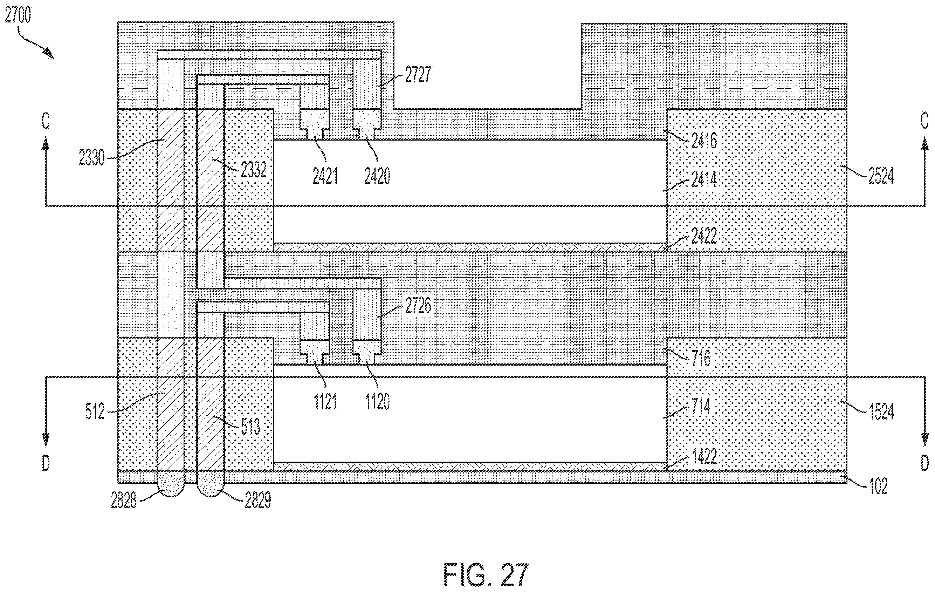

FIG. 27 illustrates a cross-section of another exemplary packaged ultrasound-on-chip device 2700, in accordance with certain embodiments described herein. The ultrasound-on-chip device 2700 is the same as ultrasound-on-chip device 2600, except that the ultrasound-on-chip device 2700 includes the RDL 2726 and 2727 instead of the RDL 1926 and 2526. Additionally, for simplicity, certain common components of the ultrasound-on-chip devices 2600 and 2700 are illustrated in different locations in FIGs. 26 and 27.

The redistribution layer (RDL) 2726 is formed above the conductive pillars 513 and 512, the contacts 1120 and 1121, and the insulating layer 716. The RDL 2726 extends through the openings formed in the insulating layer 716 to the conductive pillar 513, the conductive pillar 512, the contact 1121, and the contact 1120, and extends between the conductive pillar 513 and the contact 1121. Thus, the RDL 2726 may electrically connect the contact 1121 to the conductive pillar 513, and thereby electrically connect the conductive pillar 513 through the RDL 2726 and the contact 1121 to integrated circuitry in the first integrated circuit substrate 714. The RDL 2726 may include metal traces and vias, may be formed using electroplating (including formation of a seed layer not shown), and may include metal such as aluminum, copper, tungsten, and/or alloys of these metals. The RDL 2726 may be formed by forming metal traces, vias, and insulating material in multiple steps. Additionally, the RDL 2726 may be considered a multilayer RDL in that the RDL 2726 includes multiple horizontal layers (e.g., of metal) one above another.

The RDL 2727 is formed between the conductive pillar 2330 and the contact 2420 and between the conductive pillar 2332 and the contact 2421. Thus, the RDL 2727 may electrically connect the conductive pillar 2330 through the contact 2420 to integrated circuitry in the second integrated circuit substrate 2414, and may electrically connect the conductive pillar 2332 through the contact 2421 to integrated circuitry in the second integrated circuit substrate 2414. Further description of the RDL 2726 and 2727 and their fabrication may be found with reference to the previous description of the RDL 1926 and 2526. It should be appreciated that the RDL 2726 and 2727 may include more portions than shown. For example, there may be more conductive pillars than illustrated to which the RDL 2726 and 2727 may connect.

The ultrasound-on-chip device 2700 includes two separate integrated circuit substrates, the first integrated circuit substrate 714 and the second integrated circuit substrate 2414, as well as packaging. The first integrated circuit substrate 714 and the second integrated circuit 2414 may be considered to be arranged in a vertical stack (where a vertical stack does not preclude certain elements interposing between the two integrated circuit substrates). The first integrated circuit substrate 714 may be considered to be on one level and the second integrated circuit substrate 2414 may be considered to be on another level. The packaging of the ultrasound-on-chip device includes the conductive pillars 512, 513, 2330, and 2332, the RDL 2726 and 2727, and the solder balls 2828 and 2829. In some embodiments, the solder balls 2828 and 2829 may be coupled to a printed circuit board (PCB). In some embodiments, the solder balls 2828 and 2829 may be coupled to a heat sink. In some embodiments, the solder balls 2828 and 2829 may be coupled to an interposer that is coupled to a PCB. In some embodiments, the interposer may also function as a heat sink.

It should be appreciated that the packaging may facilitate electrical communication between an external electronic device that is electrically coupled to the solder ball 2828, such as an electronic device on a PCB that is electrically coupled (in some embodiments, through a heat sink and/or interposer) to the solder ball 2828, and integrated ultrasound circuitry (not shown) in the second integrated circuit substrate 2414. In particular, electrical communication may occur through the solder ball 2828, the conductive pillar 512, the RDL 2726, the conductive pillar 2330, the RDL 2727, the contact 2420, and the second integrated circuit substrate 2414. It should also be appreciated that the packaging may facilitate electrical communication between an external electronic device that is electrically coupled to the solder ball 2829, such as an electronic device on a PCB that is electrically coupled (in some embodiments, through a heat sink and/or interposer) to the solder ball 2829, and integrated ultrasound circuitry (not shown) in the first integrated circuit substrate 714. In particular, electrical communication may occur through the solder ball 2829, the conductive pillar 513, the RDL 2726, the contact 1121, and the first integrated circuit substrate 714. It should also be appreciated that the packaging may facilitate electrical communication between the first integrated circuit substrate 714 and the second integrated circuit substrate 2414. In particular, electrical communication may occur through the first integrated circuit substrate 714, the contact 1120, the RDL 2726, the conductive pillar 2332, the RDL 2727, the contact 2421, and the second integrated circuit substrate 2414. Thus, if a single PCB is coupled to the solder balls 2828 and 2829, the packaging may facilitate electrical communication between the single PCB and both the first and second integrated circuit substrates 714 and 2414 in the vertical stack, as well as facilitating electrical communication between the first and second integrated circuit substrates 714 and 2414 in the vertical stack. In some embodiments, electrical communication between the first integrated circuit substrate 714 and the second integrated circuit substrate 2414 may occur through the first integrated circuit substrate 714, the contact 2420, the RDL 2727, the conductive pillar 2330, the RDL 2726, the conductive pillar 512, the solder ball 2828, a PCB coupled to the solder balls 2828 and 2829, the solder ball 2829, the conductive pillar 513, the RDL 2726, the contact 1121, and the second integrated circuit substrate 2414. It should be appreciated that as described with reference to FIG. 26, electrical communication between contacts and integrated ultrasound circuitry in integrated circuit substrates may occur through bonding pads and vias (not shown).

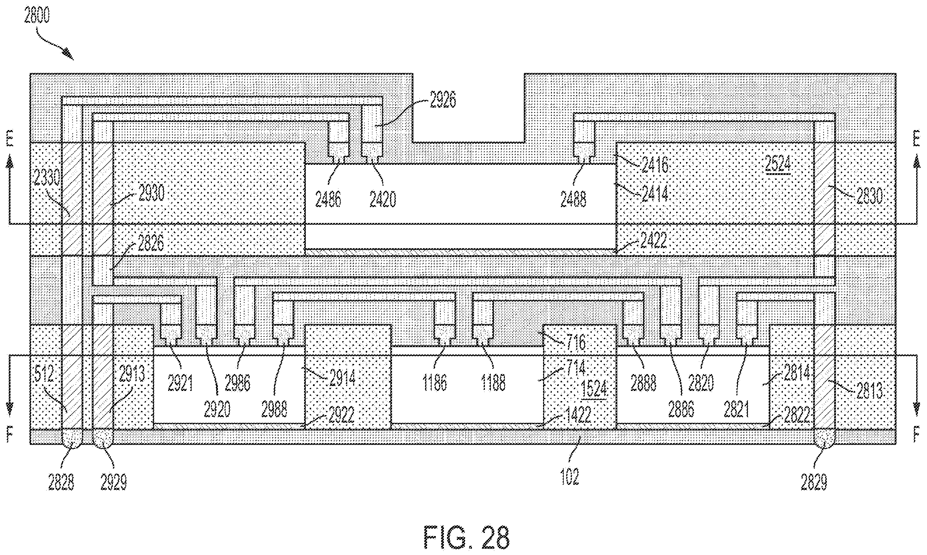

FIGs. 28-29 illustrate cross-sections of another exemplary packaged ultrasound-on-chip device 2800, in accordance with certain embodiments described herein. In the cross-section of FIG. 28, one level (the bottom level as illustrated in FIG. 28) includes the first integrated circuit substrate 714, as well as a third integrated circuit substrate 2814 and a fourth integrated circuit substrate 2914. The other level (the top level as illustrated in FIG. 28) includes the second integrated circuit substrate 2414. The first integrated circuit 714 includes contacts 1186 and 1188. The second integrated circuit includes the contact 2420 and contacts 2488 and 2490. The third integrated circuit 2814 includes contacts 2821, 2820, 2886, and 2888, and a die-attach film 2822. The fourth integrated circuit 2914 includes contacts 2921, 2920, 2986, and 2988, and a die-attach film 2822. A conductive pillar 2813 and the RDL 2826 electrically couples a solder 2828 to the contact and RDL 2826 electrically couples a solder ball 2829 to the contact 2821 on the third integrated circuit substrate 2814. A conductive pillar 2913 and RDL 2826 electrically couples a solder ball 2929 to the contact 2921 on the fourth integrated circuit substrate 2914. The RDL 2826 electrically couples the contact 2886 on the third integrated circuit substrate 2814 to the contact 2986 on the fourth integrated circuit substrate 2914. The RDL 2826 electrically couples the contact 2888 on the third integrated circuit substrate 2814 to the contact 1188 on the first integrated circuit substrate 714. The RDL 2826 electrically couples the contact 2988 on the fourth integrated circuit substrate 2914 to the contact 1186 on the first integrated circuit substrate 714. The RDL 2826, a conductive pillar 2830, and RDL 2926 electrically couples the contact 2820 on the third integrated circuit substrate 2814 to the contact 2488 on the second integrated circuit substrate 2414. The RDL 2826, a conductive pillar 2930, and the RDL 2926 electrically couples the contact 2920 on the fourth integrated circuit substrate 2914 to the contact 2486 on the second integrated circuit substrate 2414. The conductive pillar 512, the RDL 2826, the conductive pillar 2330, and the RDL 2926 electrically couples the solder ball 2828 to the contact 2420 on the second integrated circuit substrate 2414.

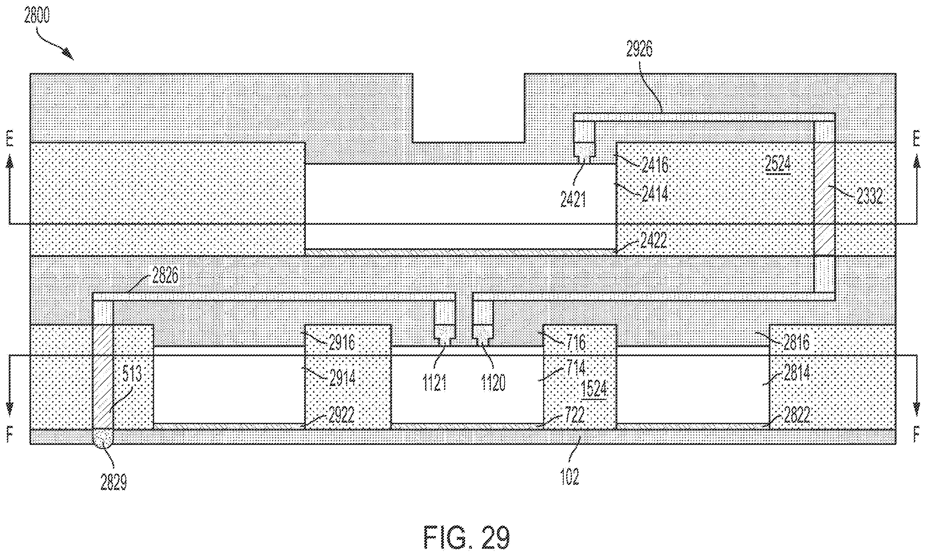

FIG. 29 illustrates another cross-section of the ultrasound-on-chip 2800 that is parallel to the cross-section of FIG. 28. The conductive pillar 513 and the RDL 2826 electrically couples the solder ball 2829 to the contact 1121 on the first integrated circuit substrate 714. The RDL 2826 and the conductive pillar 2332 electrically couples the contact 1120 on the first integrated circuit substrate 714 to the contact 2421 on the second integrated circuit substrate 2414. Thus, it should be appreciated from FIGs. 28-29 that each of the first integrated circuit substrate 714, the second integrated circuit substrate 2414, the third integrated circuit substrate 2814, and the fourth integrated circuit substrate 2914 is electrically coupled through one or more conductive pillars and/or RDLs to each other integrated circuit substrate, thus facilitating communication between each of the integrated circuit substrates. Furthermore, each of the first integrated circuit substrate 714, the second integrated circuit substrate 2414, the third integrated circuit substrate 2814, and the fourth integrated circuit substrate 2914 is electrically coupled through one or more conductive pillars and/or RDLs to solder balls that facilitate communication between each of the integrated circuit substrates to an external device (e.g., a PCB). It should be appreciated that as described with reference to FIG. 26, electrical communication between contacts and integrated ultrasound circuitry in integrated circuit substrates may occur through bonding pads and vias (not shown). Further description of integrated circuit substrates, contacts, conductive pillars, RDL, solder balls, and packaging these elements into an ultrasound-on-chip device may be found with reference to the processes illustrated in FIGs. 1-33.

As described below, the third integrated circuit substrate 2814 and the fourth integrated circuit substrate 2914 may include, for example, circuitry for wireless communication, power management, temperature sensing, global positioning, and/or inertial measurement. In some embodiments, the first integrated circuit substrate 714 may be smaller in size than the second integrated circuit substrate 2414. For example, the second integrated circuit substrate 2414, which includes ultrasonic transducers, may be large in size in order to facilitate collection of ultrasound data from a sufficiently large portion of a subject. With the difference in size between the first integrated circuit substrate 714 and the second integrated circuit substrate 2414, there may be room within the packaged ultrasound-on-chip device 2800 on the level of the first integrated circuit substrate 714 for the third integrated circuit substrate 2814 and the fourth integrated circuit substrate 2914, rather than placing these integrated circuit substrates elsewhere in the ultrasound device (e.g., on a PCB to which the ultrasound-on-chip device 2800 is coupled). This may help make the ultrasound device compact, and placing integrated circuit substrates closer together may help reduce routing parasitics and degradation of performance (speed, noise, power, etc.). Furthermore, implementing circuitry for different functions (e.g., ultrasound functions, wireless communication, power management, temperature sensing, global positioning, and/or inertial measurement) in different integrated circuit substrates may be helpful for choosing a technology process that is optimized for the particular function. It should be appreciated from FIGs. 28-29 that in some embodiments a vertical stack may include different numbers of integrated circuit substrates in different levels and may include two more integrated circuit substrates in one level.

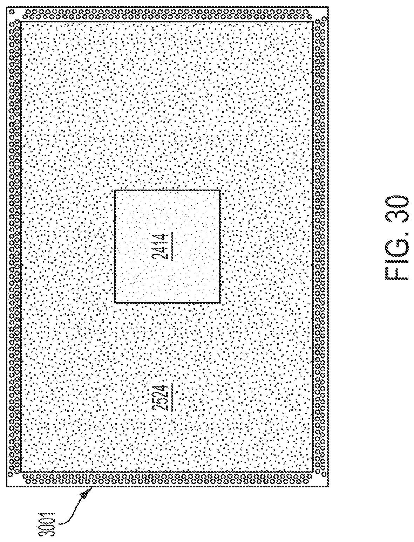

FIG. 30 illustrates a cross-section of an exemplary ultrasound-on-chip device, in accordance with certain embodiments described herein. The cross-section of FIG. 30 may be a top view of the ultrasound-on-chip device 2600 along the axis A-A, and/or a top view of the ultrasound-on-chip device 2700 along the axis C-C, and/or a top view of the ultrasound-on-chip device 2800 along the axis E-E. FIG. 30 illustrates the second integrated circuit substrate 2414, the encapsulation 2524, and conductive pillars 3001. The conductive pillars 2330, 2332, 2830, and 2930 may be among the conductive pillars 3001. The conductive pillars 3001 surround the second integrated circuit substrate 2414.

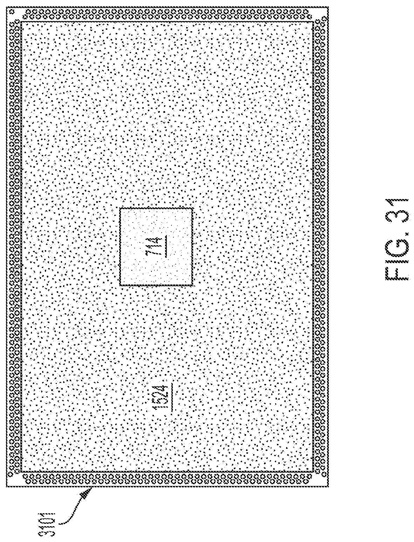

FIG. 31 illustrates a cross-section of an exemplary ultrasound-on-chip device, in accordance with certain embodiments described herein. The cross-section of FIG. 31 may be a top view of the ultrasound-on-chip device 2600 along the axis B-B and/or a top view of the ultrasound-on-chip device 2700 along the axis D-D. FIG. 31 illustrates the first integrated circuit substrate 714, the encapsulation 1524, and conductive pillars 3101. The conductive pillars 512 and 513 may be among the conductive pillars 3101. The conductive pillars 3101 surround the first integrated circuit substrate 714.

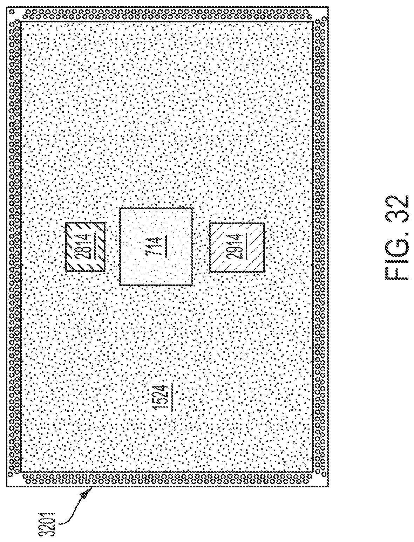

FIG. 32 illustrates a cross-section of an exemplary ultrasound-on-chip device, in accordance with certain embodiments described herein. The cross-section of FIG. 32 may be a top view of the ultrasound-on-chip device 2800 along the axis F-F. FIG. 32 illustrates the first integrated circuit substrate 714, the third integrated circuit substrate 2814, the fourth integrated circuit substrate 2914, the encapsulation 1524, and conductive pillars 3201. The conductive pillars 512, 513, 2813, and 2913 may be among the conductive pillars 3201. The conductive pillars 3201 surround the first integrated circuit substrate 714, the third integrated circuit substrate 2814, and the fourth integrated circuit substrate 2914.

It should be appreciated that a packaged ultrasound-on-chip device may include two levels, each level including one or more integrated circuit substrates. The packaging may facilitate electrically communication between each integrated circuit substrate, whether on the same level or on different levels. For example, each integrated circuit substrate may be electrically coupled through one or more conductive pillars and/or RDLs to each other integrated circuit substrate, whether on the same level as well as the other level. Furthermore, the packaging may facilitate electrically communication between each integrated circuit substrate and an external device. For example, each of the integrated circuit substrates may be electrically coupled through one or more conductive pillars and/or RDLs to solder balls that may be electrically coupled to an external device (e.g., a PCB).

In some embodiments, one integrated circuit substrate (e.g., the second integrated circuit substrate 2414) may include ultrasound transducers, ultrasound transmit circuitry (e.g., pulsers), analog ultrasound receive circuitry, and analog-to-digital converters (ADCs). In some embodiments, one integrated circuit substrate (e.g., the first integrated circuit substrate 714) may include digital ultrasound receive circuitry. In some embodiments, other integrated circuit substrates in the packaged ultrasound-on-chip device (e.g., the third and fourth integrated circuit substrates 2814 and 2914) may include circuitry for wireless communication (e.g., Bluetooth or WiFi), power management (e.g., include high-voltage transistors for a DC-DC converter), temperature sensing, global positioning, and/or inertial measurement (e.g., including one or more accelerometers, gyroscopes, and/or magnetometers). In some embodiments (e.g., if there are only two integrated circuit substrates) circuitry for wireless communication, power management, temperature sensing, global positioning, and/or inertial measurement may be incorporated into the integrated circuit substrate that includes ultrasound transducers, ultrasound transmit circuitry, analog ultrasound receive circuitry, and ADCs and/or the integrated circuit substrate that includes digital ultrasound receive circuitry. For example, the integrated circuit substrate that includes ultrasound transducers, ultrasound transmit circuitry, analog ultrasound receive circuitry, and ADCs may include wireless communication circuitry and the integrated circuit substrate that includes digital ultrasound receive circuitry may include power management circuitry. Further description of the integrated circuit substrates may be found with reference to FIGs. 33-34. In some embodiments, integrated circuit substrates on the same level may be from wafers of the same size (e.g., either 20.32 cm (8 inches ) or 30.48 cm (12 inches)) However, in some embodiments, integrated circuit substrates on the same level may be from wafers of different sizes.

FIG. 33 illustrates afunctional block diagram of an exemplary ultrasound-on-chip device 3300, in accordance with certain embodiments described herein. FIG. 33 also illustrates a printed circuit board (PCB) 3378. The ultrasound-on-chip device 3300 includes a first device 3302 and a second device 3306. The ultrasound-on-chip device 3300 may be an example of the ultrasound-on-chip devices 2600, 2700, or 2800. The first device 3302 may be an example of the second integrated circuit substrate 2414 described above, and the second device 3306 may be an example of the first integrated circuit substrate 714 described above. The first device 3302 and the second device 3306 may each be dies that are packaged together to form the ultrasound-on-chip device 3300. The first device 3302 and the second device 3306 may be application-specific integrated circuits (ASICs). The first device 3302 includes a plurality of elements 3358 (which may also be considered pixels). While only four elements 3358 are shown in FIG. 33, it should be appreciated that many more elements 3358 may be included, such as hundreds, thousands, or tens of thousands of elements. Each of the elements 3358 includes an ultrasonic transducer 3360, a pulser 3364, a receive switch 3362, an analog receive circuitry 3310 block, and an analog-to-digital converter (ADC) 3312. The first device 3302 includes the ultrasonic transducers 3360, the pulsers 3364, the receive switches 3362, the analog receive circuitry 3310, the ADCs 3312, SERDES transmit circuitry 3352, power circuitry 3348, clocking circuitry 3324, sequencing circuitry 3328, control circuitry 3326, and communication circuitry 3322. The second device 3306 includes SERDES receive circuitry 3354, digital receive circuitry 3376, power circuitry 3372, clocking circuitry 3332, sequencing circuitry 3336, control circuitry 3334, communication circuitry 3330, memory circuitry 3340, peripheral management circuitry 3338, monitoring circuitry 3374, and processing circuitry 3356. A communication link 3350 electrically connects the SERDES transmit circuitry 3352 in the first device 3302 to the SERDES receive circuity 3354 in the second device 3306. A communication link 3370 electrically connects the communication circuitry 3322 in the first device 3302 to the communication circuitry 3330 in the second device 3306. A communication link 3382 electrically connects the communication circuitry 3322 in the first device 3302 to the PCB 3378. A communication link 3384 electrically connects the communication circuitry 3330 in the second device 3306 to the PCB 3378.

A pulser 3364 may be configured to output a driving signal to an ultrasonic transducer 3360. The pulser 3364 may receive a waveform from a waveform generator (not shown) and be configured to output a driving signal corresponding to the received waveform. When the pulser 3364 is driving the ultrasonic transducer 3360 (the "transmit phase"), the receive switch 3362 may be open such that the driving signal is not applied to receive circuitry (e.g., the analog receive circuitry 3310).

The ultrasonic transducer 3360 may be configured to emit pulsed ultrasonic signals into a subject, such as a patient, in response to the driving signal received from the pulser 3364. The pulsed ultrasonic signals may be back-scattered from structures in the body, such as blood cells or muscular tissue, to produce echoes that return to the ultrasonic transducer 3360. The ultrasonic transducer 3360 may be configured to convert these echoes into electrical signals. When the ultrasonic transducer 3360 is receiving the echoes (the "receive phase"), the receive switch 3362 may be closed such that the ultrasonic transducer 3360 may transmit the electrical signals representing the received echoes through the receive switch 3362 to the analog receive circuitry 3310. Example ultrasonic transducers 3360 include capacitive micromachined ultrasonic transducers (CMUTs) and piezoelectric micromachined ultrasonic transducers (PMUTs). For example, CMUTs may include cavities formed in a substrate with a membrane/membranes overlying the cavity. The ultrasonic transducers may be arranged in an array (e.g., one-dimensional or two-dimensional).

The analog receive circuitry 3310 may include, for example, one or more analog amplifiers, one or more analog filters, analog beamforming circuitry, analog dechirp circuitry, analog quadrature demodulation (AQDM) circuitry, analog time delay circuitry, analog phase shifter circuitry, analog summing circuitry, analog time gain compensation circuitry, and/or analog averaging circuitry. The analog output of the analog receive circuitry 3310 is outputted to the ADC 3312 for conversion to a digital signal. The digital output of the ADC 3312 is outputted to the SERDES transmit circuitry 3352.

The SERDES transmit circuitry 3352 may be configured to convert parallel digital output of the ADC 3312 to a serial digital stream and to output the serial digital stream at a high-speed (e.g., 2-5 gigabits/second) over the communication link 3350. The SERDES receive circuitry 3354 may be configured to convert the serial digital stream received from the communication link 3350 to a parallel digital output and to output this parallel digital output to the digital receive circuitry 3376. The communication link 3350 may be implemented through one or more contacts, RDLs, and conductive pillars forming a conductive path between the first device 3302 and the second device 3306. For example, in the ultrasound-on-chip device illustrated in FIG. 26, the communication link 3350 may be implemented through the contact 1120, the RDL 1926, the conductive pillar 2332, the RDL 2526, and the contact 2420. As another example, in the ultrasound-on-chip device illustrated in FIG. 27, the communication link 3350 may be implemented through the contact 1120, the RDL 2726, the conductive pillar 2332, the RDL 2727, and the contact 2421.

In the ultrasound-on-chip device 3300, one block of SERDES transmit circuitry 3352 receives data from multiple ADC's 3312 and is electrically coupled, through the communication link 3350, to one block of SERDES receive circuitry 3354 that is coupled to the digital receive circuitry 3376. There may be multiple instances of SERDES transmit circuitry 3352, communication link 3350, and SERDES receive circuitry 3354, each receiving data from multiple ADC's 3312. In some embodiments, there may be one instance of SERDES transmit circuitry 3352, communication link 3350, and SERDES receive circuitry 3354 per ADC 3312 and/or per ultrasonic transducer 3360, or more generally, per element 3358. In some embodiments, there may be approximately equal to or between 1-100 parallel instances of SERDES transmit circuitry 3352, communication link 3350, and SERDES receive circuitry 3354. In some embodiments, there may be approximately equal to or between 1-10,000 parallel instances of SERDES transmit circuitry 3352, communication link 3350, and SERDES receive circuitry 3354. The data offload rate of all the parallel instances of SERDES transmit circuitry 3352, communication link 3350, and SERDES receive circuitry 3354 may make the ultrasound-on-chip device 3300 acoustically limited, meaning that it may not be necessary to insert undesired time between collection of frames of ultrasound data to offload data from the ultrasound-on-chip device 3300. The data offload rate may facilitate high pulse repetition intervals (e.g., greater than or equal to approximately 10kHz).

In some embodiments, the SERDES receive circuitry 3354 may include a mesochronous receiver. In some embodiments, the SERDES receive circuitry 3354 may include a digital phase-locked loop (PLL), a digital clock and data recovery circuit, and an equalizer. In some embodiments, the PLL of the SERDES receive circuitry 3354 may use fast on/off techniques that allow the PLL to power down and conserve power when the ultrasound-on-chip device 3300 is not generating data, and power up to full operating within an acceptably fast period of time when the ultrasound-on-chip device 3300 begins to generate data again. For further description of fast on/off techniques, see Wei, Da, et al., "A 10-Gb/s/ch, 0.6-pJ/bit/mm Power Scalable Rapid-ON/OFF Transceiver for On-Chip Energy Proportional Interconnects," I5 Journal of Solid-State Circuits 53.3 (2018): 873-883. In some embodiments, implementing the third device in an advanced technology node (e.g., 90 nm, 80 nm, 65 nm, 55 nm, 45 nm, 40 nm, 32 nm, 28 nm, 22 nm, 20 nm, 16 nm, 14 nm, 10 nm, 7 nm, 5 nm, 3 nm, etc.) may facilitate the SERDES receive circuitry 3354 operating at a high data rate (e.g., 2-5 gigabits/second).

The digital receive circuitry 3376 may include, for example, one or more digital filters, digital beamforming circuitry, digital quadrature demodulation (DQDM) circuitry, averaging circuitry, digital dechirp circuitry, digital time delay circuitry, digital phase shifter circuitry, digital summing circuitry, digital multiplying circuitry, requantization circuitry, waveform removal circuitry, image formation circuitry, backend processing circuitry and/or one or more output buffers. The image formation circuitry in the digital receive circuitry 3376 may be configured to perform apodization, back projection and/or fast hierarchy back projection, interpolation range migration (e.g., Stolt interpolation) or other Fourier resampling techniques, dynamic focusing techniques, delay and sum techniques, tomographic reconstruction techniques, Doppler calculation, frequency and spatial compounding, and/or low and high-pass filtering, etc.

Referring to the first device 3302, the communication circuitry 3322 in the first device 3302 may be configured to provide communication between the first device 3302 and the second device 3306 over the communication link 3370 (or more than one communication links 3370). The communication circuitry 3322 may facilitate communication of signals from any circuitry on the first device 3302 to the second device 3306 and/or communication of signals from any circuitry on the second device 3306 to the first device 3302 (aside from communication facilitated by the SERDES transmit circuitry 3352, the communication link 3350, and the SERDES receive circuitry 3354). The communication link 3370 may be implemented through one or more contacts, RDLs, and conductive pillars forming a conductive path between the first device 3302 and the second device 3306. In the example ultrasound-on-chip device illustrated in FIG. 26, the communication link 3370 may be implemented through the contact 1120, the RDL 1926, the conductive pillar 2332, the RDL 2526, and the contact 2420. In the example ultrasound-on-chip device illustrated in FIG. 27, the communication link 3370 may be implemented through the contact 1120, the RDL 2726, the conductive pillar 2332, the RDL 2727, and the contact 2421.

The communication circuitry 3322 in the first device 3302 may also be configured to provide communication between the first device 3302 and the PCB 3378 over the communication link 3382 (or more than one communication links 3382). The communication circuitry 3322 may facilitate communication of signals from any circuitry on the first device 3302 to the PCB 3378 and/or communication of signals from any circuitry on the PCB 3378 to the first device 3302. For example, the PCB 3378 may provide control signals to the first device 3302 through the communication link 3382 and the communication circuitry 3322 that may then be used by the control circuitry 3326. The communication link 3382 may be implemented through one or more solder balls, contacts, RDLs, and conductive pillars forming a conductive path between the first device 3302 and an external device. For example, in the ultrasound-on-chip device illustrated in FIG. 26, the communication link 3382 may be implemented through the solder ball 2828, the conductive pillar 512, the RDL 1926, the conductive pillar 2330, the RDL 2526, and the contact 2421. As another example, in the ultrasound-on-chip device illustrated in FIG. 27, the communication link 3382 may be implemented through the solder ball 2828, the conductive pillar 512, the RDL 2726, the conductive pillar 2330, the RDL 2727, and the contact 2421.

The clocking circuitry 3324 in the first device 3302 may be configured to generate some or all of the clocks used in the first device 3302 and/or the second device 3306. In some embodiments, the clocking circuitry 3324 may receive a high-speed clock (e.g., a 1.5625 GHz or a 2.5 GHz clock) from an external source that the clocking circuitry 3324 may feed to various circuit components of the ultrasound-on-chip device 3300. In some embodiments, the clocking circuitry 3324 may divide and/or multiply the received high-speed clock to produce clocks of different frequencies (e.g., 20 MHz, 40 MHz, 100 MHz, or 200 MHz) that the clocking circuitry 3324 may feed to various components of the ultrasound-on-chip device 3300. In some embodiments, the clocking circuitry 3324 may separately receive two or more clocks of different frequencies, such as the frequencies described above.

The control circuitry 3326 in the first device 3302 may be configured to control various circuit components in the first device 3302. For example, the control circuitry 3326 may control and/or parameterize the pulsers 3364, the receive switches 3362, the analog receive circuitry 3310, the ADCs 3312, the SERDES transmit circuitry 3352, the power circuitry 3348, the communication circuitry 3322, the clocking circuitry 3324, the sequencing circuitry 3328, digital waveform generators, delay meshes, and/or time-gain compensation circuitry (the latter three of which are not shown in FIG. 33). The control circuitry 3326 may also be configured to control any circuitry on the second device 3306.

The sequencing circuitry 3328 in the first device 3302 may be configured to coordinate various circuit components on the first device 3302 that may or may not be digitally parameterized. In some embodiments, the sequencing circuitry 3328 may control the timing and ordering of parameter changes in the first device 3302 and/or the second device 3306, control triggering of transmit and receive events, and control data flow (e.g., from the first device 3302 to the second device 3306). In some embodiments, the sequencing circuitry 3328 may control execution of an imaging sequence which may be specific to the selected imaging mode, preset, and user settings. In some embodiments, the sequencing circuitry 3328 in the first device 3302 may be configured as a master sequencer that triggers events on sequencing circuitry 3336 in the second device 3306 that is configured as a slave sequencer and has been digitally parameterized. In some embodiments, the sequencing circuitry 3336 in the second device 3306 is configured as a master sequencer that triggers events on the sequencing circuitry 3328 in the first device 3302 that is configured as a slave sequencer and has been digitally parameterized. In some embodiments, the sequencing circuitry 3328 in the first device 3302 is configured to control parameterized circuit components on both the first device 3302 and the second device 3306. In some embodiments, the sequencing circuitry 3328 in the first device 3302 and the sequencing circuitry 3336 in the second device 3306 may operate in synchronization by using a clock derived from the same source (e.g., provided by the clocking circuitry).

The power circuitry 3348 in the first device 3302 may include low dropout regulators, switching power supplies, and/or DC-DC converters to supply the first device 3302 and/or the second device 3306. In some embodiments, the power circuitry 3348 may include multi-level pulsers and/or charge recycling circuitry. For further description of multi-level pulsers and charge recycling circuitry, see U.S. Patent No. 9,492,144 titled "MULTI-LEVEL PULSER AND RELATED APPARATUS AND METHODS," granted on November 15, 2016, and U.S. Patent App. No. 15/087,914 titled "MULTILEVEL BIPOLAR PULSER," issued as U.S. Patent No. 10,082,565, each of which is assigned to the assignee of the instant application.

The second device 3306 additionally includes communication circuitry 3330, clocking circuitry 3332, control circuitry 3334, sequencing circuitry 3336, peripheral management circuitry 3338, memory circuitry 3340, power circuitry 3372, processing circuitry 3356, and monitoring circuitry 3374. The communication circuitry 3330 in the second device 3306 may be configured to provide communication between the second device 3306 and the first device 3302 over the communication link 3370 (or more than one communication links 3370). The communication circuitry 3330 may facilitate communication of signals from any circuitry on the second device 3306 to the first device 3302 and/or communication of signals from any circuitry on the first device 3302 to the second device 3306.

The communication circuitry 3330 in the second device 3306 may also be configured to provide communication between the second device 3306 and the PCB 3378 over the communication link 3384 (or more than one communication links 3384). The communication circuitry 3330 may facilitate communication of signals from any circuitry on the second device 3306 to the PCB 3378 and/or communication of signals from any circuitry on the PCB 3378 to the second device 3306. For example, the PCB 3378 may provide control signals to the second device 3306 through the communication link 3384 and the communication circuitry 3330 that may then be used by the control circuitry 3334. The communication link 3384 may be implemented through one or more solder balls, contacts, RDLs, and conductive pillars forming a conductive path between the second device 3306 and an external device. For example, in the ultrasound-on-chip device illustrated in FIG. 26, the communication link 3384 may be implemented through the solder ball 2829, the conductive pillar 513, the RDL 1926, and the contact 1121. As another example, in the example ultrasound-on-chip device illustrated in FIG. 27, the communication link 3384 may be implemented through the solder ball 2829, the conductive pillar 513, the RDL 2726, and the contact 1121.

The clocking circuitry 3332 in the second device 3306 may be configured to generate some or all of the clocks used in the second device 3306 and/or the first device 3302. In some embodiments, the clocking circuitry 3332 may receive a high-speed clock (e.g., a 1.5625 GHz or a 2.5 GHz clock) that the clocking circuitry 3332 may feed to various circuit components of the ultrasound-on-chip device 3300. In some embodiments, the clocking circuitry 3332 may divide and/or multiply the received high-speed clock to produce clocks of different frequencies (e.g., 20 MHz, 40 MHz, 100 MHz, or 200 MHz) that the clocking circuitry 3332 may feed to various components. In some embodiments, the clocking circuitry 3332 may separately receive two or more clocks of different frequencies, such as the frequencies described above.

The control circuitry 3334 in the second device 3306 may be configured to control various circuit components in the second device 3306. For example, the control circuitry 3334 may control and/or parameterize the SERDES receive circuitry 3354, the digital receive circuitry 3376, the communication circuitry 3330, the clocking circuitry 3332, the sequencing circuitry 3336, the peripheral management circuitry 3338, the memory circuitry 3340, the power circuitry 3372, and the processing circuitry 3356. The control circuitry 3334 may also be configured to control any circuitry on the first device 3302.

The sequencing circuitry 3336 in the second device 3306 may be configured to coordinate various circuit components on the second device 3306 that may or may not be digitally parameterized. In some embodiments, the sequencing circuitry 3336 in the second device 3306 is configured as a master sequencer that triggers events on the sequencing circuitry 3328 in the first device 3302 that has been digitally parameterized. In some embodiments, the sequencing circuitry 3328 in the first device 3302 is configured as a master sequencer that triggers events on the sequencing circuitry 3336 in the first device 3302 that is configured as a slave sequencer and has been digitally parameterized. In some embodiments, the sequencing circuitry 3336 in the second device 3306 is configured to control parameterized circuit components on both the first device 3302 and the second device 3306. In some embodiments, the sequencing circuitry 3336 in the second device 3306 and the sequencing circuitry 3328 in the first device 3302 may operate in synchronization by using a clock derived from the same source (e.g., provided by the clocking circuitry).

The peripheral management circuitry 3338 may be configured to generate a high-speed serial output data stream. For example, the peripheral management circuitry 3338 may be a Universal Serial Bus (USB) 2.0, 3.0, or 3.1 module. The peripheral management circuitry 3338 may additionally or alternatively be configured to allow an external microprocessor to control various circuit components of the ultrasound-on-chip device 3300 over a USB connection. As another example, the peripheral management circuitry 3338 may include a WiFi module or a module for controlling another type of peripheral. In some embodiments, this high-speed serial output data stream may be outputted to the PCB 3378.

The memory circuitry 3340 may be configured to buffer and/or store digitized image data (e.g., image data produced by imaging formation circuitry and/or other circuitry in the digital receive circuitry 3376). For example, the memory circuitry 3340 may be configured to enable the ultrasound-on-chip device 3300 to retrieve image data in the absence of a wireless connection to a remote server storing the image data. Furthermore, when a wireless connection to a remote server is available, the memory circuitry 3340 may also be configured to provide support for wireless connectivity conditions such as lossy channels, intermittent connectivity, and lower data rates, for example. In addition to storing digitized image data, the memory circuitry 3340 may also be configured to store timing and control parameters for synchronizing and coordinating operation of elements in the ultrasound-on-chip device 3300. The power circuitry 3372 may include power supply amplifiers for supplying power to the second device 3306.

The processing circuitry 3356, which may be in the form of one or more embedded processors, may be configured to perform processing functions. In some embodiments, the processing circuitry 3356 may be configured to perform sequencing functions, either for the first device 3302 or for the second device 3306. For example, the processing circuitry 3356 may control the timing and ordering of parameter changes in the first device 3302 and/or the second device 3306, control triggering of transmit and receive events, and/or control data flow (e.g., from the first device 3302 to the second device 3306). In some embodiments, the processing circuitry 3356 may control execution of an imaging sequence which may be specific to the selected imaging mode, preset, and user settings. In some embodiments, the processing circuitry 3356 may perform external system control, such as controlling the peripheral management circuitry 3338, the processing circuitry 3356, controlling power sequencing (e.g., for the power circuitry 3348 and/or the power circuitry 3372), and interfacing with the monitoring circuitry 3374. In some embodiments, the processing circuitry 3356 may perform internal system control, such as configuring data flow within the chip (e.g., from the first device 3302 to the second device 3306), calculating or controlling the calculation of processing and image formation parameters (e.g., for image formation circuitry), controlling on chip clocking (e.g., for the clocking circuitry 3324 and/or the clocking circuitry 3332), and/or controlling power (e.g., for the power circuitry 3348 and/or the power circuitry 3372). The processing circuitry 3356 may be configured to perform functions described above as being performed by other components of the ultrasound-on-chip device 3300, and in some embodiments certain components described herein may be absent if their functions are performed by the processing circuitry 3356.

The monitoring circuitry 3374 may include, but is not limited to, temperature monitoring circuitry (e.g., thermistors), power measurement circuitry (e.g., voltage and current sensors), nine-axis motion circuitry (e.g., gyroscopes, accelerometers, compasses), battery monitoring circuitry (e.g., coulomb counters), and/or circuitry checking for status or exception conditions of other on-board circuits (e.g., power controllers, protection circuitry, etc.).

It should be understood that there may be many more instances of each component shown in FIG. 33. For example, there may be hundreds, thousands, or tens of thousands of ultrasonic transducers 3360, pulsers 3364, receive switches 3362, analog receive circuitry 3310 blocks, SERDES transmit circuitry 3352 blocks, SERDES receive circuitry 3354 blocks, and/or digital receive circuitry 3376 blocks. Additionally, it should be understood that certain components shown in FIG. 33 may receive signals from more components than shown or transmit signals to more components than shown (e.g., in a multiplexed fashion, or after averaging). For example, a given pulser 3364 may output signals to one or more ultrasonic transducers 3360, a given receive switch 3362 may receive signals from one or more ultrasonic transducers 3360, a given block of analog receive circuitry 3310 may receive signals from one or more receive switches 3362, a given ADC 3312 may receive signals from one or more blocks of analog receive circuitry 3310, a given block of SERDES transmit circuitry 3352 may receive signals from one or more ADCs 3312. In some embodiments, a given ultrasound element may have an ultrasonic transducer 3360 and a dedicated pulser 3364, receive switch 3362, analog receive circuitry 3310 block, ADC 3312, and/or SERDES transmit circuitry 3352 block. It should also be understood that certain embodiments of an ultrasound-on-chip device may have more or fewer components than shown in FIG. 33.

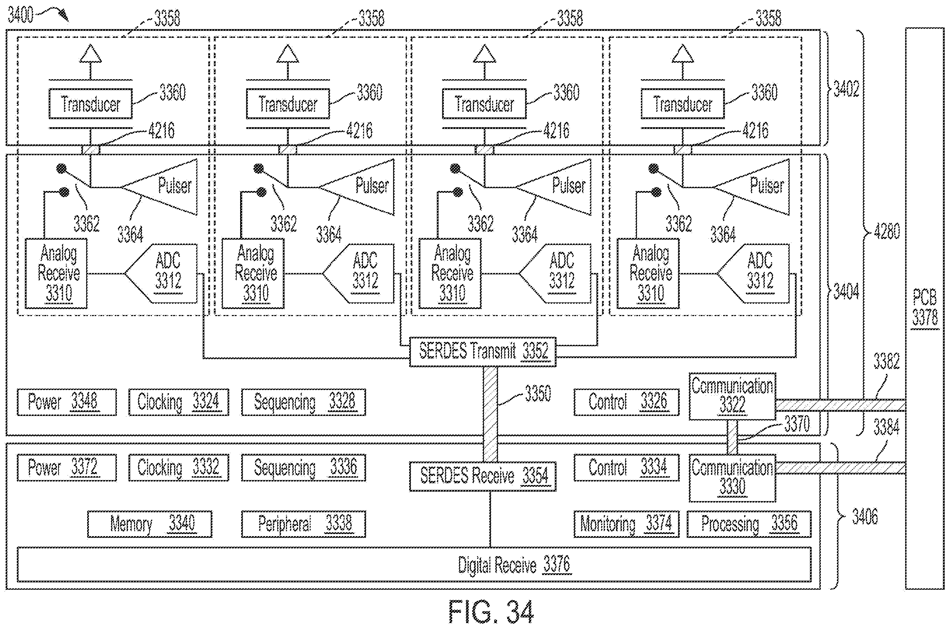

FIG. 34 illustrates another example functional block diagram of another exemplary ultrasound-on-chip 3400, in accordance with certain embodiments described herein. FIG. 34 also illustrates the printed circuit board (PCB) 3378. The ultrasound-on-chip device 3400 may be an example of the ultrasound-on-chip devices 2600, 2700, or 2800. The ultrasound-on-chip device 3400 is the same as the ultrasound-on-chip device 3300, except that the ultrasound-on-chip device 3400 includes a first device 3402, a second device 3404, and a third device 3406. The combination 3480 of the first device 3402 and the second device 3404 may be an example of the second integrated circuit substrate 2414 described above, and the third device 3406 may be an example of the first integrated circuit substrate 714 described above. The first device 3402 includes the ultrasound transducers 3360 that are included in the first device 3302 of the ultrasound-on-chip device 3400. The second device 3404 includes the remaining circuitry that is included in the first device 3302 of the ultrasound-on-chip device 3400. The third device 3406 is the same as the second device 3306 of the ultrasound-on-chip device 3300. The first device 3402, the second device 3404, and the third device 3406 may each be dies that are packaged together to form the ultrasound-on-chip device 3400. The second device 3404 and the third device 3406 may be application-specific integrated circuits (ASICs). The first device 3402 and the second device 3404 are bonded together at bonding points 3416. The bonding between the first device 3402 and the second device 3404 may include, for example, thermal compression (also referred to herein as "thermocompression"), eutectic bonding, silicide bonding (which is a bond formed by bringing silicon of one substrate into contact with metal on a second substrate under sufficient pressure and temperature to form a metal silicide, creating a mechanical and electrical bond), or solder bonding. The bonding points 3416 electrically connect the ultrasonic transducers 3360 in the first device 3402 to the pulsers 3364 and the receive switches 3362 in the second device 3404.

The pulser 3364 may be configured to output a driving signal to an ultrasonic transducer 3360 through a bonding point 3416. When the ultrasonic transducer 3360 is receiving the echoes, the receive switch 3362 may be closed such that the ultrasonic transducer 3360 may transmit the electrical signals representing the received echoes through the bonding point 3416 and the receive switch 3362 to the analog receive circuitry 3310.

For further description of circuit components of ultrasound-on-chip devices (e.g., the ultrasound-on-chip devices 3300 and 3400), see U.S. Patent No. 9,521,991 titled "MONOLITHIC ULTRASONIC IMAGING DEVICES, SYSTEMS, AND METHODS," granted on December 20, 2016 (and assigned to the assignee of the instant application). For further description of fabricating ultrasound-on-chip devices (e.g., the ultrasound-on-chip devices 3300 and 3400), see U.S. Patent No. 9,067,779 titled "MICROFABRICATED ULTRASONIC TRANSDUCERS AND RELATED APPARATUS AND METHODS," granted on June 30, 2015 (and assigned to the assignee of the instant application); and see U.S. Patent Application Publication No. 2019/0275561 titled "ULTRASOUND TRANSDUCER DEVICES AND METHODS FOR FABRICATING ULTRASOUND TRANSDUCER DEVICES," filed on March 8, 2019 (and assigned to the assignee of the instant application).

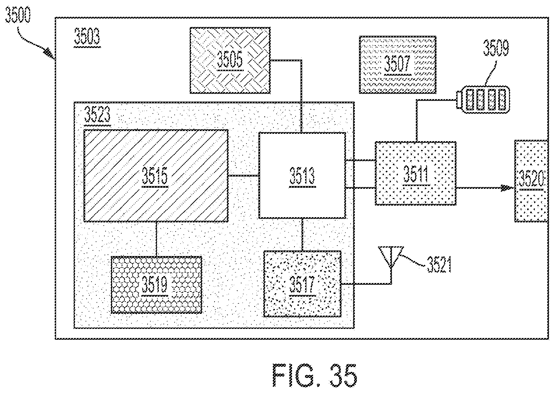

FIG. 35 illustrates a functional block diagram of an exemplary ultrasound device 3500, in accordance with certain embodiments described herein. The ultrasound device 3500 may be a wearable ultrasound device. The ultrasound device 3500 includes a PCB 3503 to which is coupled an ultrasound-on-chip device 3523, memory 3505, a power management chip (PMIC) 3507, a battery 3509, a charger and switch 3511, an antenna 3521, and an output port 3520. The ultrasound-on-chip device 3523 includes a digital signal processing (DSP) chip 3513, a sensor chip 3515, a wireless communication chip 3517, and a PMIC 3519. The memory 3505 and the DSP chip 3513 are electrically coupled together. The sensor chip 3515 and the DSP chip 3513 are electrically coupled together. The sensor chip 3515 and the PMIC chip 3519 are electrically coupled together. The DSP chip 3513 and the wireless communication chip 3517 are electrically coupled together. The wireless communication chip 3517 and the antenna 3521 are coupled together. The battery 3509 and the charger and switch 3511 are electrically coupled together. The charger and switch 3511 and the output port 3520 are electrically coupled together.

The PCB 3503 may be, for example, the PCB 3378. The memory 3505 may be, for example, DRAM. The antenna 3521 may be, for example, a 2.4 GHz antenna. The output port 3520 may be, for example, a USB port. The ultrasound-on-chip device 3523 may include, for example, the ultrasound-on-chip devices 2600, 2700, 2800, 3300, or 3400. The DSP chip 3513 may include, for example, the second integrated circuit substrate 2414, the second device 3306, and/or the third device 3406. The sensor chip 3515 may include, for example, the second integrated circuit substrate 2414, the first device 3302, and/or the combination 3480 of the first device 3402 and the third device 3404. The wireless communication chip 3517 may be, for example, a Bluetooth or WiFi communication chip, and may include, for example, either the third or fourth integrated circuit substrates 2814 or 2914. The PMIC 3519 may include, for example, high-voltage transistors for a DC-DC converter, and may include, for example, either the third or fourth integrated circuit substrates 2814 or 2914.

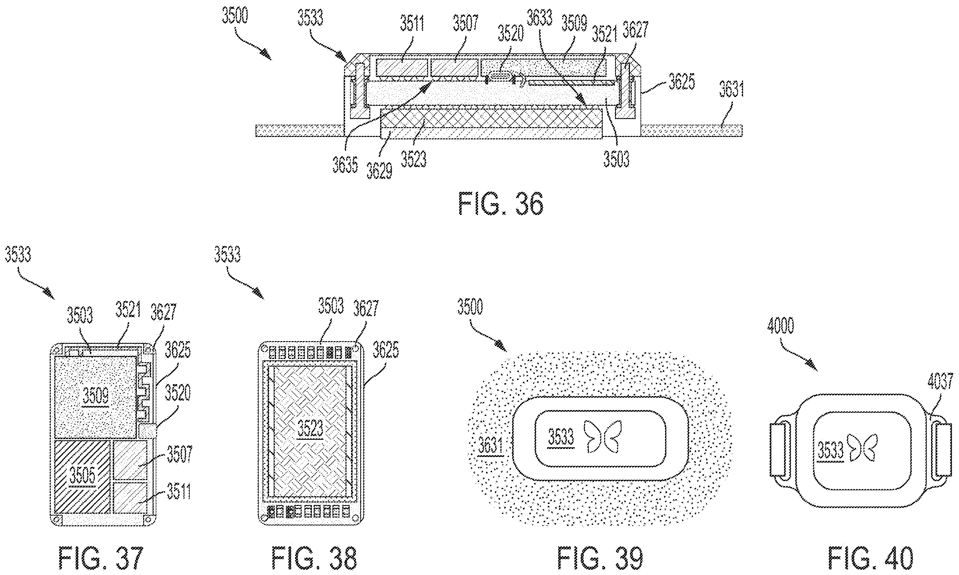

FIG. 36 illustrates a schematic diagram of a side view of the ultrasound device 3500, in accordance with certain embodiments described herein. The ultrasound device 3500 includes an ultrasound module 3533 and a patch 3631. The ultrasound module 3533 includes the PCB 3503, the ultrasound-on-chip device 3523, the memory 3505, the PMIC 3507, the battery 3509, the charger and switch 3511, the antenna 3521, the output port 3520, a housing 3625, screws 3627, an acoustic lens 3629, solder balls 3633, and solder balls 3635. The housing 3625 encloses internal components of the ultrasound device 3500. The screws 3627 couple the PCB 3503 (and, thereby, components coupled to the PCB 3503) to the housing 3625. The solder balls 3633 couple the ultrasound-on-chip device 3523 to the PCB 3503 and thereby facilitate electrical communication between the ultrasound-on-chip device 3523 and the PCB 3503. The solder balls 3633 may include, for example, the solder balls 2828, 2829, 2829, and/or 2929. The solder balls 3635 couple the charger and switch 3511 and the PMIC 3507 to the PCB and thereby facilitate electrical communication between the charger and switch 3511 and the PMIC 3507 and the PCB 3503. The acoustic lens 3629 may be configured for impedance matching and signal attenuation. The patch 3631 may include adhesive material and may be configured to couple the ultrasound device 3500 to the skin of a patient. In some embodiments, the patch 3631 may not be included, and the ultrasound device 3500 may be coupled to the patient by other means, such as a strap.

FIG. 37 illustrates a schematic diagram of a top view of the ultrasound module 3533, in accordance with certain embodiments described herein. FIG. 37 illustrates the PCB 3503, the memory 3505, the PMIC 3507, the battery 3509, the charger and switch 3511, the antenna 3521, the output port 3520, the housing 3625, and the screws 3627.

FIG. 38 illustrates a schematic diagram of a bottom view of the ultrasound module 3533, in accordance with certain embodiments described herein. FIG. 38 illustrates the PCB 3503, the ultrasound-on-chip device 3523, the housing 3625, and the screws 3627.

FIG. 39 illustrates a top view of the ultrasound device 3500, in accordance with certain embodiments described herein. The ultrasound device 3500 includes the ultrasound module 3533 and the patch 3631.

FIG. 40 illustrates a top view of another ultrasound device 4000, in accordance with certain embodiments described herein. The ultrasound device 4000 includes the ultrasound module 3533 and holes 4037. A strap (now shown in figure) may be threaded through the holes, and the strap may extend around a portion of a subject (e.g., around a subject's abdomen) and thereby couple to the subject. The strap may therefore be an alternative to the patch 3631 for coupling the ultrasound device 4000 to the subject.

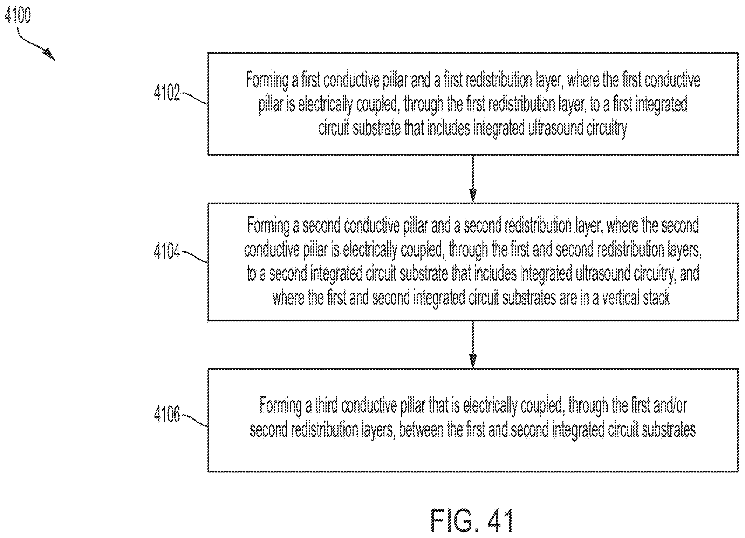

FIG. 41 illustrates an example process 4100 for packaging an ultrasound-on-chip device, in accordance with certain embodiments described herein. In act 4102, a first conductive pillar (e.g., the conductive pillar 513) and a first redistribution layer (RDL) (e.g., the RDL 1926, 2726, or 2826) are formed. The first conductive pillar is electrically coupled, through the first RDL, to a first integrated circuit substrate (e.g., the first integrated circuit substrate 714) that includes integrated ultrasound circuitry. In some embodiments, electrical coupling between the first conductive pillar and the first integrated circuit substrate may further occur through a contact (e.g., the contact 1121) on the first integrated circuit substrate.

In act 4104, a second conductive pillar (e.g., the conductive pillar 512 and/or the conductive pillar 2330) and a second redistribution layer (RDL) (e.g., the RDL 2526, 2727, or 2926) are formed. The second conductive pillar is electrically coupled, through the first RDL and/or the second RDL, to a second integrated circuit substrate (e.g., the second integrated circuit substrate 2414) that includes integrated ultrasound circuitry. The first and second integrated circuit substrates are in a vertical stack. In some embodiments, the second conductive pillar may be electrically coupled to the second integrated circuit substrate through the first RDL but not the second RDL. In some embodiments, the second conductive pillar may be electrically coupled to the second integrated circuit substrate through the second RDL but not the first RDL. In some embodiments, electrical coupling between the second conductive pillar and the second integrated circuit substrate may further occur through a contact (e.g., the contact 2420) on the second integrated circuit substrate.

In act 4106, a third conductive pillar (e.g., the conductive pillar 2332) is formed. The third conductive pillar is electrically coupled, through the first RDL and the second RDL, between the first and second integrated circuit substrates. In some embodiments, electrical coupling between the first and second integrated circuit substrate may further occur through a contact (e.g., the contact 1120) on the first integrated circuit substrate and a contact (e.g., the contact 2421) on the second integrated circuit substrate. In some embodiments, act 4106 may be omitted. For example, communication between the first and second integrated circuit substrates may occur through the first and second conductive pillars, and may be routed through a printed circuit board (PCB) which is electrically coupled to both the first and second conductive pillars.

Various aspects of the present disclosure may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Various inventive concepts may be embodied as one or more processes, of which an example has been provided. The acts performed as part of each process may be ordered in any suitable way. Thus, embodiments may be constructed in which acts are performed in an order different than illustrated, which may include performing some acts simultaneously, even though shown as sequential acts in illustrative embodiments. Further, one or more of the processes may be combined and/or omitted, and one or more of the processes may include additional steps.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

As used herein, reference to a numerical value being between two endpoints should be understood to encompass the situation in which the numerical value can assume either of the endpoints. For example, stating that a characteristic has a value between A and B, or between approximately A and B, should be understood to mean that the indicated range is inclusive of the endpoints A and B unless otherwise noted.

The terms "approximately" and "about" may be used to mean within ±20% of a target value in some embodiments, within ±10% of a target value in some embodiments, within ±5% of a target value in some embodiments, and yet within ±2% of a target value in some embodiments. The terms "approximately" and "about" may include the target value.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

Having described above several aspects of at least one embodiment, it is to be appreciated various alterations, modifications, and improvements will readily occur to those skilled in the art, , insofar as they fall within the scope of the invention as set forth in the accompanying claims. Accordingly, the foregoing description and drawings are by way of example only.

## Claims

1. An ultrasound-on-chip device (2600; 2700; 2800; 3300; 3400), comprising:
a first integrated circuit substrate (714; 3306) comprising first integrated ultrasound circuitry (2655), the first integrated circuit substrate (714; 3306) having a first surface;
a first conductive pillar (513) disposed adjacent the first integrated circuit substrate (714; 3306) and extending substantially along a first direction;
a first redistribution layer (1926; 2726; 2826) adjacent the first surface of the first integrated circuit substrate (714; 3306) and electrically coupling the first integrated ultrasound circuitry (2655) to the first conductive pillar (513);
a second integrated circuit substrate (2414; 3302) comprising second integrated ultrasound circuitry (2645), the second integrated circuit substrate (2414; 3302) having a first surface and a second surface opposite the first surface, the first and second integrated circuit substrates (714, 2414; 3302) being stacked along the first direction so that the first surface of the second integrated circuit substrate (2414; 3302) is adjacent the first surface of the first integrated circuit substrate (714; 3306);
a second conductive pillar (512; 2330) disposed adjacent the second integrated circuit substrate (2414; 3302) and extending substantially along the first direction;
a third conductive pillar (2332) disposed adjacent the second integrated circuit substrate (2414; 3302) and extending substantially along the first direction, wherein a communication link (3350) between serial-deserializer (SerDes) transmit circuitry (3352) on the second integrated circuit substrate and SerDes receive circuitry (3354) on the first integrated circuit substrate is implemented through the third conductive pillar (2332);
a second redistribution layer (2526; 2727; 2926) adjacent the second surface of the second integrated circuit substrate (2414; 3302) and electrically coupling the second integrated ultrasound circuitry (2645) to the second conductive pillar (512; 2330) and the third conductive pillar (2332), wherein
the first redistribution layer (1926; 2726; 2826) is a multilayer redistribution layer that includes:
a first layer that electrically couples the first integrated ultrasound circuitry (2655) to the first conductive pillar (513); and
a second layer that electrically couples the first integrated ultrasound circuitry (2655) to the third conductive pillar (2332);
the third conductive pillar (2332) electrically couples the second layer of the first redistribution layer (1926; 2726; 2826) to the second redistribution layer (2526; 2727; 2926); and
a printed circuit board (PCB) (3378), wherein the first integrated circuit substrate (714; 3306) is disposed between the PCB (3378) and the second integrated circuit substrate (2414; 3302);
wherein the first conductive pillar electrically couples the PCB (3378) to the first integrated circuit substrate (714; 3306) and the second conductive pillar (512; 2330) couples the PCB (3378) to the second integrated circuit substrate (2414; 3302).

2. The ultrasound-on-chip device (2600; 2700; 2800; 3300; 3400) of claim 1, wherein the second integrated circuit substrate (2414) further comprises ultrasonic transducers (2647) coupled to the second integrated ultrasound circuitry (2645).

3. The ultrasound-on-chip device (3300) of claim 1, wherein
the first integrated ultrasound circuitry (2655) of the first integrated circuit substrate (714; 3306) comprises digital receive circuitry (3376); and
the second integrated ultrasound circuitry (2645) of the second integrated circuit substrate (2414; 3302) comprises:
a pulser (3364);
a receive switch (3362);
analog receive circuitry (3310); and
an analog-to-digital converter (3312),
the first redistribution layer (1926; 2726; 2826) electrically couples the first conductive pillar (513) to the digital receive circuitry (3376), and
the second redistribution layer (2526; 2727; 2926) electrically couples the second conductive pillar (512; 2330) to the analog-to-digital converter (3312).

4. The ultrasound-on-chip device (3300) of claim 3, wherein
the second integrated circuit substrate (2414; 3302) further comprises an ultrasound transducer electrically (3360) coupled to the pulser (3364).

5. The ultrasound-on-chip device (3400) of claim 1, wherein
the first integrated ultrasound circuitry (2655) of the first integrated circuit substrate (714; 3306) comprises digital receive circuitry (3376),
the second integrated circuit (2414; 3302) substrate comprises a first device (3402) and a second device (3404) bonded together,
the first device (3402) comprises an ultrasound transducer (3360), and
the second integrated ultrasound circuitry (2645) is on the second device (3404) and comprises:
a pulser (3364);
a receive switch (3362);
analog receive circuitry (3310); and
an analog-to-digital converter (3312),
the first redistribution layer (1926; 2726; 2826) electrically couples the first conductive pillar (513) to the digital receive circuitry (3376) and
the second redistribution layer (2526; 2727; 2926) electrically couples the second conductive pillar (512; 2330) to the second device (3404).

6. The ultrasound-on-chip device of claim 1, further comprising:
a fourth conductive pillar disposed adjacent the first integrated circuit substrate (714; 3306), wherein
the first redistribution layer (1926; 2726; 2826) couples the second conductive pillar (512; 2330) to the fourth conductive pillar.

7. The ultrasound-on-chip device of claim 6, wherein the second and fourth conductive pillars are stacked to one another along the first direction.

8. A method, comprising:
obtaining a first integrated circuit substrate (714; 3306) comprising first integrated ultrasound circuitry (2655) and obtaining a second integrated circuit substrate (2414; 3302) comprising second integrated ultrasound circuitry (2645);
forming a first conductive pillar (513) adjacent the first integrated circuit substrate (714; 3306) and extending substantially along a first direction;
forming a first redistribution layer (1926; 2726; 2826) adjacent a first surface of the first integrated circuit substrate (714; 3306) and electrically coupling the first conductive pillar (513) to the first integrated ultrasound circuitry (2655);
stacking the first and second integrated circuit substrates (714; 3306, 2414; 3302) to one another along the first direction so that the first surface of the first integrated circuit substrate (714; 3306) is adjacent a first surface of the second integrated circuit substrate (2414; 3302);
forming a second conductive pillar (512; 2330) adjacent the second integrated circuit substrate (2414; 3302) and extending substantially along the first direction;
prior to forming a second redistribution layer (2526; 2727; 2926), forming a third conductive pillar (2332) adjacent the second integrated circuit substrate (2414; 3302) and extending substantially along the first direction, wherein forming the second redistribution layer (2526; 2727; 2926) comprises electrically coupling serial-deserializer (SerDes) transmit circuitry (3352) on the second integrated circuit substrate to SerDes receive circuitry (3354) on the first integrated circuit substrate; and
after forming the third conductive pillar (2332), forming the second redistribution layer (2526; 2727; 2926) adjacent a second surface of the second integrated circuit substrate (2414; 3302) opposite the first surface of the second integrated circuit substrate (2414; 3302) and electrically coupling the second conductive pillar (512; 2330) and the third conductive pillar (2332) to the second integrated ultrasound circuitry (2645), wherein
the first redistribution layer (1926; 2726; 2826) is formed as a multilayer redistribution layer that includes:
a first layer that electrically couples the first integrated ultrasound circuitry (2655) to the first conductive pillar (513); and
a second layer that electrically couples the first integrated ultrasound circuitry (2655) to the third conductive pillar (2332);
the third conductive pillar (2332) electrically couples the second layer of the first redistribution layer (1926; 2726; 2826) to the second redistribution layer (2526; 2727; 2926); and
placing the first integrated circuit substrate (714; 3306) on a printed circuit board (PCB) (3378) so that the first conductive pillar (513) electrically couples the PCB (3378) to the first integrated circuit substrate and the second conductive pillar (512) electrically couples the PCB (3378) to the second integrated circuit substrate.

9. The method of claim 8, wherein
the first integrated ultrasound circuitry (2655) of the first integrated circuit substrate (714; 3306) comprises digital receive circuitry (3376), and
the second integrated ultrasound circuitry (2645) of the second integrated circuit substrate (2414; 3302) comprises:
a pulser (3364);
a receive switch (3362);
analog receive circuitry(3310); and
an analog-to-digital converter (3312),
forming the first redistribution layer (1926; 2726; 2826) comprises electrically coupling the first conductive pillar (513) to the digital receive circuitry (3376), and
forming the second redistribution layer (2526; 2727; 2926) comprises electrically coupling the second conductive pillar (512; 2330) to the analog-to-digital converter (3312).

10. The method of claim 8, wherein
the first integrated ultrasound circuitry (2655) of the first integrated circuit substrate (714; 3306) comprises digital receive circuitry (3376),
the second integrated circuit substrate (2414; 3302) comprises a first device (3402) and a second device (3404) bonded together,
the first device (3402) comprises an ultrasound transducer (3360), and
the second integrated ultrasound circuitry (2645) is on the second device (3404) and comprises:
a pulser (3364),
a receive switch (3362),
analog receive circuitry (3310), and
an analog-to-digital converter (3312),
forming the first redistribution layer (1926; 2726; 2826) comprises electrically coupling the first conductive pillar (513) to the digital receive circuitry (3376), and
forming the second redistribution layer (2526; 2727; 2926) comprises electrically coupling the second conductive pillar (512; 2330) to the second device (3404).

## Patentansprüche

1. Ultraschall-on-Chip-Vorrichtung (2600; 2700; 2800; 3300; 3400), umfassend:
ein erstes Substrat mit integrierter Schaltung (714; 3306), das eine erste integrierte Ultraschallschaltung (2655) umfasst, wobei das erste Substrat mit integrierter Schaltung (714; 3306) eine erste Oberfläche aufweist;
eine erste elektrisch leitfähige Säule (513), die benachbart zu dem ersten Substrat mit integrierter Schaltung (714; 3306) angeordnet ist und sich im Wesentlichen entlang einer ersten Richtung erstreckt;
eine erste Umverdrahtungsschicht (1926; 2726; 2826), die benachbart zu der ersten Oberfläche des ersten Substrats mit integrierter Schaltung (714; 3306) ist und die erste integrierte Ultraschallschaltung (2655) elektrisch mit der ersten elektrisch leitfähigen Säule (513) koppelt;
ein zweites Substrat mit integrierter Schaltung (2414; 3302), das eine zweite integrierte Ultraschallschaltung (2645) umfasst, wobei das zweite Substrat mit integrierter Schaltung (2414; 3302) eine erste Oberfläche und eine der ersten Oberfläche gegenüberliegende zweite Oberfläche aufweist, wobei das erste und das zweite Substrat mit integrierter Schaltung (714, 2414; 3302) entlang der ersten Richtung gestapelt sind, so dass die erste Oberfläche des zweiten Substrats mit integrierter Schaltung (2414; 3302) benachbart zu der ersten Oberfläche des ersten Substrats mit integrierter Schaltung (714; 3306) ist;
eine zweite elektrisch leitfähige Säule (512; 2330), die benachbart zu dem zweiten Substrat mit integrierter Schaltung (2414; 3302) angeordnet ist und sich im Wesentlichen entlang der ersten Richtung erstreckt;
eine dritte elektrisch leitfähige Säule (2332), die benachbart zu dem zweiten Substrat mit integrierter Schaltung (2414; 3302) angeordnet ist und sich im Wesentlichen entlang der ersten Richtung erstreckt, wobei eine Kommunikationsverbindung (3350) zwischen einer Seriell-Deserialisierer-(SerDes)-Sendeschaltung (3352) auf dem zweiten Substrat mit integrierter Schaltung und einer SerDes-Empfangsschaltung (3354) auf dem ersten Substrat mit integrierter Schaltung durch die dritte elektrisch leitfähige Säule (2332) implementiert ist;
eine zweite Umverdrahtungsschicht (2526; 2727; 2926), die benachbart zu der zweiten Oberfläche des zweiten Substrats mit integrierter Schaltung (2414; 3302) ist und die zweite integrierte Ultraschallschaltung (2645) elektrisch mit der zweiten elektrisch leitfähigen Säule (512; 2330) und der dritten elektrisch leitfähigen Säule (2332) koppelt, wobei
die erste Umverdrahtungsschicht (1926; 2726; 2826) eine mehrschichtige Umverdrahtungsschicht ist, die Folgendes beinhaltet:
eine erste Schicht, die die erste integrierte Ultraschallschaltung (2655) elektrisch mit der ersten elektrisch leitfähigen Säule (513) koppelt; und
eine zweite Schicht, die die erste integrierte Ultraschallschaltung (2655) elektrisch mit der dritten elektrisch leitfähigen Säule (2332) koppelt;
die dritte elektrisch leitfähige Säule (2332) die zweite Schicht der ersten Umverdrahtungsschicht (1926; 2726; 2826) elektrisch mit der zweiten Umverdrahtungsschicht (2526; 2727; 2926) koppelt; und
eine Leiterplatte (PCB) (3378), wobei das erste Substrat mit integrierter Schaltung (714; 3306) zwischen der PCB (3378) und dem zweiten Substrat mit integrierter Schaltung (2414; 3302) angeordnet ist;
wobei die erste elektrisch leitfähige Säule die PCB (3378) elektrisch mit dem ersten Substrat mit integrierter Schaltung (714; 3306) koppelt und die zweite elektrisch leitfähige Säule (512; 2330) die PCB (3378) mit dem zweiten Substrat mit integrierter Schaltung (2414; 3302) koppelt.

2. Ultraschall-on-Chip-Vorrichtung (2600; 2700; 2800; 3300; 3400) nach Anspruch 1, wobei das zweite Substrat mit integrierter Schaltung (2414) ferner Ultraschallwandler (2647) umfasst, die mit der zweiten integrierten Ultraschallschaltung (2645) gekoppelt sind.

3. Ultraschall-on-Chip-Vorrichtung (3300) nach Anspruch 1, wobei
die erste integrierte Ultraschallschaltung (2655) des ersten Substrats mit integrierter Schaltung (714; 3306) eine digitale Empfangsschaltung (3376) umfasst; und
die zweite integrierte Ultraschallschaltung (2645) des zweiten Substrats mit integrierter Schaltung (2414; 3302) Folgendes umfasst:
einen Impulsgeber (3364);
einen Empfangsschalter (3362);
eine analoge Empfangsschaltung (3310); und
einen Analog-Digital-Wandler (3312),
die erste Umverdrahtungsschicht (1926; 2726; 2826) die erste elektrisch leitfähige Säule (513) elektrisch mit der digitalen Empfangsschaltung (3376) koppelt und
die zweite Umverdrahtungsschicht (2526; 2727; 2926) die zweite elektrisch leitfähige Säule (512; 2330) elektrisch mit dem Analog-Digital-Wandler (3312) koppelt.

4. Ultraschall-on-Chip-Vorrichtung (3300) nach Anspruch 3, wobei
das zweite Substrat mit integrierter Schaltung (2414; 3302) ferner einen Ultraschallwandler (3360) umfasst, der elektrisch mit dem Impulsgeber (3364) gekoppelt ist.

5. Ultraschall-on-Chip-Vorrichtung (3400) nach Anspruch 1, wobei
die erste integrierte Ultraschallschaltung (2655) des ersten Substrats mit integrierter Schaltung (714; 3306) eine digitale Empfangsschaltung (3376) umfasst,
das zweite Substrat mit integrierter Schaltung (2414; 3302) eine erste Vorrichtung (3402) und eine zweite Vorrichtung (3404) umfasst, die miteinander gebondet sind,
die erste Vorrichtung (3402) einen Ultraschallwandler (3360) umfasst und
die zweite integrierte Ultraschallschaltung (2645) sich auf der zweiten Vorrichtung (3404) befindet und Folgendes umfasst:
einen Impulsgeber (3364);
einen Empfangsschalter (3362);
eine analoge Empfangsschaltung (3310); und
einen Analog-Digital-Wandler (3312),
die erste Umverdrahtungsschicht (1926; 2726; 2826) die erste elektrisch leitfähige Säule (513) elektrisch mit der digitalen Empfangsschaltung (3376) koppelt und
die zweite Umverdrahtungsschicht (2526; 2727; 2926) die zweite elektrisch leitfähige Säule (512; 2330) elektrisch mit der zweiten Vorrichtung (3404) koppelt.

6. Ultraschall-on-Chip-Vorrichtung nach Anspruch 1, ferner umfassend:
eine vierte elektrisch leitfähige Säule, die benachbart zu dem ersten Substrat mit integrierter Schaltung (714; 3306) angeordnet ist, wobei
die erste Umverdrahtungsschicht (1926; 2726; 2826) die zweite elektrisch leitfähige Säule (512; 2330) mit der vierten elektrisch leitfähigen Säule koppelt.

7. Ultraschall-on-Chip-Vorrichtung nach Anspruch 6, wobei die zweite und die vierte elektrisch leitfähige Säule entlang der ersten Richtung aufeinander gestapelt sind.

8. Verfahren, umfassend:
Erhalten eines ersten Substrats mit integrierter Schaltung (714; 3306), das eine erste integrierte Ultraschallschaltung (2655) umfasst, und Erhalten eines zweiten Substrats mit integrierter Schaltung (2414; 3302), das eine zweite integrierte Ultraschallschaltung (2645) umfasst;
Ausbilden einer ersten elektrisch leitfähigen Säule (513), die benachbart zu dem ersten Substrat mit integrierter Schaltung (714; 3306) ist und sich im Wesentlichen entlang einer ersten Richtung erstreckt;
Ausbilden einer ersten Umverdrahtungsschicht (1926; 2726; 2826), die benachbart zu einer ersten Oberfläche des ersten Substrats mit integrierter Schaltung (714; 3306) ist, und elektrisches Koppeln der ersten elektrisch leitfähigen Säule (513) mit der ersten integrierten Ultraschallschaltung (2655); Stapeln des ersten und des zweiten Substrats mit integrierter Schaltung (714; 3306, 2414; 3302) aufeinander entlang der ersten Richtung, so dass die erste Oberfläche des ersten Substrats mit integrierter Schaltung (714; 3306) benachbart zu einer ersten Oberfläche des zweiten Substrats mit integrierter Schaltung (2414; 3302) ist;
Ausbilden einer zweiten elektrisch leitfähigen Säule (512; 2330), die benachbart zu dem zweiten Substrat mit integrierter Schaltung (2414; 3302) ist und sich im Wesentlichen entlang der ersten Richtung erstreckt;
vor dem Ausbilden einer zweiten Umverdrahtungsschicht (2526; 2727; 2926) Ausbilden einer dritten elektrisch leitfähigen Säule (2332), die benachbart zu dem zweiten Substrat mit integrierter Schaltung (2414; 3302) ist und sich im Wesentlichen entlang der ersten Richtung erstreckt, wobei das Ausbilden der zweiten Umverdrahtungsschicht (2526; 2727; 2926) das elektrische Koppeln einer Seriell-Deserialisierer-(SerDes)-Sendeschaltung (3352) auf dem zweiten Substrat mit integrierter Schaltung mit einer SerDes-Empfangsschaltung (3354) auf dem ersten Substrat mit integrierter Schaltung umfasst; und
nach dem Ausbilden der dritten elektrisch leitfähigen Säule (2332) Ausbilden der zweiten Umverdrahtungsschicht (2526; 2727; 2926) benachbart zu einer der ersten Oberfläche des zweiten Substrats mit integrierter Schaltung (2414; 3302) gegenüberliegenden zweiten Oberfläche des zweiten Substrats mit integrierter Schaltung (2414; 3302) und elektrisches Koppeln der zweiten elektrisch leitfähigen Säule (512; 2330) und der dritten elektrisch leitfähigen Säule (2332) mit der zweiten integrierten Ultraschallschaltung (2645), wobei die erste Umverdrahtungsschicht (1926; 2726; 2826) als mehrschichtige Umverdrahtungsschicht ausgebildet wird, die Folgendes beinhaltet:
eine erste Schicht, die die erste integrierte Ultraschallschaltung (2655) elektrisch mit der ersten elektrisch leitfähigen Säule (513) koppelt; und
eine zweite Schicht, die die erste integrierte Ultraschallschaltung (2655) elektrisch mit der dritten elektrisch leitfähigen Säule (2332) koppelt;
die dritte elektrisch leitfähige Säule (2332) die zweite Schicht der ersten Umverdrahtungsschicht (1926; 2726; 2826) elektrisch mit der zweiten Umverdrahtungsschicht (2526; 2727; 2926) koppelt; und
Platzieren des ersten Substrats mit integrierter Schaltung (714; 3306) auf einer Leiterplatte (PCB) (3378), so dass die erste elektrisch leitfähige Säule (513) die PCB (3378) elektrisch mit dem ersten Substrat mit integrierter Schaltung koppelt und die zweite elektrisch leitfähige Säule (512) die PCB (3378) elektrisch mit dem zweiten Substrat mit integrierter Schaltung koppelt.

9. Verfahren nach Anspruch 8, wobei
die erste integrierte Ultraschallschaltung (2655) des ersten Substrats mit integrierter Schaltung (714; 3306) eine digitale Empfangsschaltung (3376) umfasst und
die zweite integrierte Ultraschallschaltung (2645) des zweiten Substrats mit integrierter Schaltung (2414; 3302) Folgendes umfasst:
einen Impulsgeber (3364);
einen Empfangsschalter (3362);
eine analoge Empfangsschaltung (3310); und
einen Analog-Digital-Wandler (3312),
das Ausbilden der ersten Umverdrahtungsschicht (1926; 2726; 2826) das elektrische Koppeln der ersten elektrisch leitfähigen Säule (513) mit der digitalen Empfangsschaltung (3376) umfasst und
das Ausbilden der zweiten Umverdrahtungsschicht (2526; 2727; 2926) das elektrische Koppeln der zweiten elektrisch leitfähigen Säule (512; 2330) mit dem Analog-Digital-Wandler (3312) umfasst.

10. Verfahren nach Anspruch 8, wobei
die erste integrierte Ultraschallschaltung (2655) des ersten Substrats mit integrierter Schaltung (714; 3306) eine digitale Empfangsschaltung (3376) umfasst,
das zweite Substrat mit integrierter Schaltung (2414; 3302) eine erste Vorrichtung (3402) und eine zweite Vorrichtung (3404) umfasst, die miteinander gebondet sind,
die erste Vorrichtung (3402) einen Ultraschallwandler (3360) umfasst und
die zweite integrierte Ultraschallschaltung (2645) sich auf der zweiten Vorrichtung (3404) befindet und Folgendes umfasst:
einen Impulsgeber (3364),
einen Empfangsschalter (3362),
eine analoge Empfangsschaltung (3310) und
einen Analog-Digital-Wandler (3312),
das Ausbilden der ersten Umverdrahtungsschicht (1926; 2726; 2826) das elektrische Koppeln der ersten elektrisch leitfähigen Säule (513) mit der digitalen Empfangsschaltung (3376) umfasst und
das Ausbilden der zweiten Umverdrahtungsschicht (2526; 2727; 2926) das elektrische Koppeln der zweiten elektrisch leitfähigen Säule (512; 2330) mit der zweiten Vorrichtung (3404) umfasst.

## Revendications

1. Dispositif à ultrasons sur puce (2600 ; 2700 ; 2800 ; 3300 ; 3400), comprenant :
un premier substrat de circuit intégré (714 ; 3306) comprenant un premier circuit à ultrasons intégré (2655), le premier substrat de circuit intégré (714 ; 3306) ayant une première surface ;
un premier pilier conducteur (513) disposé adjacent au premier substrat de circuit intégré (714 ; 3306) et s'étendant sensiblement le long d'une première direction ;
une première couche de redistribution (1926 ; 2726 ; 2826) adjacente à la première surface du premier substrat de circuit intégré (714 ; 3306) et couplant électriquement le premier circuit à ultrasons intégré (2655) au premier pilier conducteur (513) ;
un second substrat de circuit intégré (2414 ; 3302) comprenant un second circuit à ultrasons intégré (2645), le second substrat de circuit intégré (2414 ; 3302) ayant une première surface et une seconde surface opposée à la première surface,
les premier et second substrats de circuit intégré (714, 2414 ; 3302) étant empilés le long de la première direction de sorte que la première surface du second substrat de circuit intégré (2414 ; 3302) est adjacente à la première surface du premier substrat de circuit intégré (714 ; 3306) ;
un deuxième pilier conducteur (512 ; 2330) disposé adjacent au second substrat de circuit intégré (2414 ; 3302) et s'étendant sensiblement le long de la première direction ;
un troisième pilier conducteur (2332) disposé adjacent au second substrat de circuit intégré (2414 ; 3302) et s'étendant sensiblement le long de la première direction, dans lequel une liaison de communication (3350) entre le circuit de transmission sérialiseur-désérialiseur (SerDes) (3352) sur le second substrat de circuit intégré et le circuit de réception SerDes (3354) sur le premier substrat de circuit intégré est mise en œuvre à travers le troisième pilier conducteur (2332) ;
une seconde couche de redistribution (2526 ; 2727 ; 2926) adjacente à la seconde surface du second substrat de circuit intégré (2414 ; 3302) et couplant électriquement le second circuit à ultrasons intégré (2645) au deuxième pilier conducteur (512 ; 2330) et au troisième pilier conducteur (2332), dans lequel
la première couche de redistribution (1926 ; 2726 ; 2826) est une couche de redistribution multicouche qui comporte :
une première couche qui couple électriquement le premier circuit à ultrasons intégré (2655) au premier pilier conducteur (513) ; et
une seconde couche qui couple électriquement le premier circuit à ultrasons intégré (2655) au troisième pilier conducteur (2332) ;
le troisième pilier conducteur (2332) couple électriquement la seconde couche de la première couche de redistribution (1926 ; 2726 ; 2826) à la seconde couche de redistribution (2526 ; 2727 ; 2926) ; et
une carte de circuit imprimé (PCB) (3378), dans lequel le premier substrat de circuit intégré (714 ; 3306) est disposé entre la PCB (3378) et le second substrat de circuit intégré (2414 ; 3302) ;
dans lequel le premier pilier conducteur couple électriquement la PCB (3378) au premier substrat de circuit intégré (714 ; 3306) et le deuxième pilier conducteur (512 ; 2330) couple la PCB (3378) au second substrat de circuit intégré (2414 ; 3302).

2. Dispositif à ultrasons sur puce (2600 ; 2700 ; 2800 ; 3300 ; 3400) selon la revendication 1, dans lequel le second substrat de circuit intégré (2414) comprend en outre des transducteurs ultrasoniques (2647) couplés au second circuit à ultrasons intégré (2645).

3. Dispositif à ultrasons sur puce (3300) selon la revendication 1, dans lequel
le premier circuit à ultrasons intégré (2655) du premier substrat de circuit intégré (714 ; 3306) comprend un circuit de réception numérique (3376) ; et
le second circuit à ultrasons intégré (2645) du second substrat de circuit intégré (2414 ; 3302) comprend :
un générateur d'impulsions (3364) ;
un commutateur de réception (3362) ;
un circuit de réception analogique (3310) ; et
un convertisseur analogique-numérique (3312),
la première couche de redistribution (1926 ; 2726 ; 2826) couple électriquement le premier pilier conducteur (513) au circuit de réception numérique (3376), et
la seconde couche de redistribution (2526 ; 2727 ; 2926) couple électriquement le deuxième pilier conducteur (512 ; 2330) au convertisseur analogique-numérique (3312).

4. Dispositif à ultrasons sur puce (3300) selon la revendication 3, dans lequel
le second substrat de circuit intégré (2414 ; 3302) comprend en outre un transducteur à ultrasons (3360) couplé électriquement au générateur d'impulsions (3364).

5. Dispositif à ultrasons sur puce (3400) selon la revendication 1, dans lequel
le premier circuit à ultrasons intégré (2655) du premier substrat de circuit intégré (714 ; 3306) comprend un circuit de réception numérique (3376),
le second substrat de circuit intégré (2414 ; 3302) comprend un premier dispositif (3402) et un second dispositif (3404) liés ensemble,
le premier dispositif (3402) comprend un transducteur à ultrasons (3360), et
le second circuit à ultrasons intégré (2645) se trouve sur le second dispositif (3404) et comprend :
un générateur d'impulsions (3364) ;
un commutateur de réception (3362) ;
un circuit de réception analogique (3310) ; et
un convertisseur analogique-numérique (3312),
la première couche de redistribution (1926 ; 2726 ; 2826) couple électriquement le premier pilier conducteur (513) au circuit de réception numérique (3376) et
la seconde couche de redistribution (2526 ; 2727 ; 2926) couple électriquement le deuxième pilier conducteur (512 ; 2330) au second dispositif (3404).

6. Dispositif à ultrasons sur puce selon la revendication 1, comprenant en outre :
un quatrième pilier conducteur disposé adjacent au premier substrat de circuit intégré (714 ; 3306), dans lequel
la première couche de redistribution (1926 ; 2726 ; 2826) couple le deuxième pilier conducteur (512 ; 2330) au quatrième pilier conducteur.

7. Dispositif à ultrasons sur puce selon la revendication 6, dans lequel les deuxième et quatrième piliers conducteurs sont empilés l'un sur l'autre le long de la première direction.

8. Procédé, comprenant :
l'obtention d'un premier substrat de circuit intégré (714 ; 3306) comprenant un premier circuit à ultrasons intégré (2655) et l'obtention d'un second substrat de circuit intégré (2414 ; 3302) comprenant un second circuit à ultrasons intégré (2645) ;
la formation d'un premier pilier conducteur (513) adjacent au premier substrat de circuit intégré (714 ; 3306) et s'étendant sensiblement le long d'une première direction ;
une première couche de redistribution (1926 ; 2726 ; 2826) adjacente à une première surface du premier substrat de circuit intégré (714 ; 3306) et couplant électriquement le premier pilier conducteur (513) au premier circuit à ultrasons intégré (2655) ;
l'empilement des premier et second substrats de circuit intégré (714 ; 3306, 2414 ; 3302) l'un sur l'autre le long de la première direction de sorte que la première surface du premier substrat de circuit intégré (714 ; 3306) est adjacente à une première surface du second substrat de circuit intégré (2414 ; 3302) ;
la formation d'un deuxième pilier conducteur (512 ; 2330) adjacent au second substrat de circuit intégré (2414 ; 3302) et s'étendant sensiblement le long de la première direction ; avant la formation d'une seconde couche de redistribution (2526 ; 2727 ; 2926), la formation d'un troisième pilier conducteur (2332) adjacent au second substrat de circuit intégré (2414 ; 3302) et s'étendant sensiblement le long de la première direction, dans lequel la formation de la seconde couche de redistribution (2526 ; 2727 ; 2926) comprend le couplage électrique du circuit de transmission sérialiseur-désérialiseur (SerDes) (3352) sur le second substrat de circuit intégré au circuit de réception SerDes (3354) sur le premier substrat de circuit intégré ; et
après la formation du troisième pilier conducteur (2332), la formation de la seconde couche de redistribution (2526 ; 2727 ; 2926) adjacente à une seconde surface du second substrat de circuit intégré (2414 ; 3302) opposée à la première surface du second substrat de circuit intégré (2414 ; 3302) et le couplage électrique du deuxième pilier conducteur (512 ; 2330) et du troisième pilier conducteur (2332) au second circuit à ultrasons intégré (2645), dans lequel
la première couche de redistribution (1926 ; 2726 ; 2826) est formée en tant que couche de redistribution multicouche qui comporte :
une première couche qui couple électriquement le premier circuit à ultrasons intégré (2655) au premier pilier conducteur (513) ; et
une seconde couche qui couple électriquement le premier circuit à ultrasons intégré (2655) au troisième pilier conducteur (2332) ;
le troisième pilier conducteur (2332) couple électriquement la seconde couche de la première couche de redistribution (1926 ; 2726 ; 2826) à la seconde couche de redistribution (2526 ; 2727 ; 2926) ; et
le placement du premier substrat de circuit intégré (714 ; 3306) sur une carte de circuit imprimé (PCB) (3378) de sorte que le premier pilier conducteur (513) couple électriquement la PCB (3378) au premier substrat de circuit intégré et que le deuxième pilier conducteur (512) couple électriquement la PCB (3378) au second substrat de circuit intégré.

9. Procédé selon la revendication 8, dans lequel
le premier circuit à ultrasons intégré (2655) du premier substrat de circuit intégré (714 ; 3306) comprend un circuit de réception numérique (3376), et
le second circuit à ultrasons intégré (2645) du second substrat de circuit intégré (2414 ; 3302) comprend :
un générateur d'impulsions (3364) ;
un commutateur de réception (3362) ;
un circuit de réception analogique (3310) ; et
un convertisseur analogique-numérique (3312),
la formation de la première couche de redistribution (1926 ; 2726 ; 2826) comprend le couplage électrique du premier pilier conducteur (513) au circuit de réception numérique (3376), et la formation de la seconde couche de redistribution (2526 ; 2727 ; 2926) comprend le couplage électrique du deuxième pilier conducteur (512 ; 2330) au convertisseur analogique-numérique (3312).

10. Procédé selon la revendication 8, dans lequel
le premier circuit à ultrasons intégré (2655) du premier substrat de circuit intégré (714 ; 3306) comprend un circuit de réception numérique (3376),
le second substrat de circuit intégré (2414 ; 3302) comprend un premier dispositif (3402) et un second dispositif (3404) liés ensemble,
le premier dispositif (3402) comprend un transducteur à ultrasons (3360), et
le second circuit à ultrasons intégré (2645) se trouve sur le second dispositif (3404) et comprend :
un générateur d'impulsions (3364),
un commutateur de réception (3362),
un circuit de réception analogique (3310), et
un convertisseur analogique-numérique (3312),
la formation de la première couche de redistribution (1926 ; 2726 ; 2826) comprend le couplage électrique du premier pilier conducteur (513) au circuit de réception numérique (3376), et
la formation de la seconde couche de redistribution (2526 ; 2727 ; 2926) comprend le couplage électrique du deuxième pilier conducteur (512 ; 2330) au second dispositif (3404).
